# EUROPEAN PATENT APPLICATION

(11) **EP 3 951 794 A1**
(43) Date of publication of application: **09.02.2022**
(21) Application number: 20784555.3
(22) Date of filing: 26.03.2020
(51) Int. Cl.: G16H 50/20, A61B 5/00, A61B 10/00

(54) **SKIN DISEASE ANALYZING PROGRAM, SKIN DISEASE ANALYZING METHOD, SKIN DISEASE ANALYZING DEVICE, AND SKIN DISEASE ANALYZING SYSTEM**

(30) Priority: 29.03.2019 JP 2019067229; 15.11.2019 JP 2019206765
(71) Applicant: University of Tsukuba, Ibaraki 305-8577 (JP); Soinn Inc., Tokyo 1940004 (JP); Life Science Institute, Inc., Tokyo 100-8251 (JP)
(72) Inventor: ISHIDA, Mitsuyoshi, Tokyo 101-0047 (JP)
(74) Representative: Müller-Boré & Partner Patentanwälte PartG mbB
(86) International application number: PCT/JP2020/013684
(87) International publication number: WO 2020/203651

(57) **Abstract**

Provided are a skin disease analysis program, a skin disease analysis method, a skin disease analyzer, and a skin disease analysis system, which can analyze skin disease more accurately. The program according to the present invention is executed by a computer to execute a second step of predicting a kind of skin tumor for an image to be analyzed of skin tumor by a first-learned model that has machine learned from images of affected parts of various skin diseases in advance, and either one or both of a first step and a third step, in which the first step determines whether or not the image to be analyzed is an image of skin tumor by a skin disease determination engine, prior to the second step, and in a case where the determination result of the second step has been one kind of skin tumors that are easily mistaken for each other, the third step re-predicts the kind of skin tumor for the image to be analyzed by a second-learned model that has machine learned from images of affected parts of specific skin diseases including the skin tumors that are easily mistaken for each other.

## Description

### Technical Field

The present invention relates to a skin disease analysis program, a skin disease analysis method, a skin disease analyzer, and a skin disease analysis system.

### Background Art

Conventionally, when an abnormality is found on the skin, it is common to go to a dermatologist, an internist, or other clinic or hospital, and a doctor observes the skin of a patient with the naked eye, makes a diagnosis, and identifies the disease name. However, there has been a case where it is hardly distinguished between, for example, "lentigo" and "melanoma (malignant melanoma)" that have similar shapes to each other.

In recent years, a magnifying glass with a light source called a dermoscope has come to be used, and a doctor has become able to identify the disease name more accurately by observing fine parts of the skin with such a magnifying glass. However, such a dermoscope has not been arranged in every medical institution, and further, requires a certain degree of skill for use and specialized knowledge of dermatology. In addition, the doctor using a dermoscope and the patient are required to be physically close to each other.

In this regard, with the spread of internet and the improvement of communication speed, a mobile terminal with a camera such as a smartphone or a tablet computer has become common, and it has become extremely easy to shoot and transmit digital video. In view of such circumstances, a system for determining the disease name by analyzing an image has been developed.

For example, Patent Literature 1 discloses a system in which an image sent from a user terminal to a skin disease analysis center device is subjected to a comparative analysis with the data for skin disease analysis accumulated inside the center device, possible skin disease is predicted for the image, and the predicted disease name is proposed.

### Citation List

### Patent Literature

Patent Literature 1: JP 2005-58398 A

### Summary of Invention

### Technical Problem

However, Patent Literature 1 does not specifically indicate which disease can be determined, and does not disclose the specific accuracy, and thus, it is considered to be difficult to put the system into practical use.

The present invention has been made to solve the above problems, and an object of the present invention is to provide a skin disease analysis program, a skin disease analysis method, a skin disease analyzer, and a skin disease analysis system, which can analyze skin disease more accurately.

### Solution to Problem

In order to solve the problems described above, the present invention is a skin disease analysis program executed by a computer, and is characterized in that the program allows the computer to execute a second step of predicting the kind of skin tumor for an image of skin tumor to be analyzed by a first-learned model that has machine learned from images of affected parts of various skin diseases in advance, and either one or both of a first step and a third step, in which the first step is a step of determining whether or not the image to be analyzed is an image of skin tumor by a skin disease determination engine prior to the second step, and in a case where the determination result of the second step has been one kind of skin tumors that are easily mistaken for each other, the third step is a step of re-predicting the kind of skin tumor for the image to be analyzed by a second-learned model that has machine learned from images of affected parts of specific skin diseases including the skin tumors that are easily mistaken for each other.

Further, in order to solve the problems described above, the present invention is a skin disease analysis program executed by a computer, and is characterized in that the program allows the computer to execute a first step of determining whether or not an image to be analyzed is an image of skin tumor by a skin disease determination engine, and in a case where the image to be analyzed has been determined to be an image of skin tumor by the first step, a second step of predicting the kind of skin tumor for the image to be analyzed by a learned model that has machine learned from images of affected parts of various skin diseases in advance.

In addition, the present invention is a skin disease analysis program executed by a computer, and is characterized in that the program allows the computer to execute a second step of predicting the kind of skin tumor for an image to be analyzed by a first learned model that has machine learned from images of affected parts of skin diseases by a skin disease determination engine, and in a case where the image to be analyzed has been determined to be one kind of skin tumors that are easily mistaken for malignant melanoma by the second step, a third step of re-predicting the kind of skin tumor by a second-learned model that has machine learned from images of affected parts of diseases including skin tumors that are easily erroneously determined and malignant melanoma.

### Advantageous Effects of Invention

According to the present invention, a skin disease analysis program, a skin disease analysis method, a skin disease analyzer, and a skin disease analysis system, which can analyze skin disease more accurately, can be provided.

### Brief Description of Drawings

Fig. 1 is a block diagram showing a hardware configuration of the skin disease analysis system 1 according to one embodiment of the present invention.
Fig. 2 is a block diagram showing a functional configuration of the AI server 204 shown in Fig. 1.
Fig. 3 is a diagram illustrating an outline of operation of the skin disease analysis system 1 shown in Fig. 1.
Fig. 4 is a flowchart showing an example of the process of the skin disease analysis program executed in the skin disease analysis system 1.
Fig. 5 is a flowchart showing the process of exclusion determination executed in the skin disease analyzer 200.
Fig. 6 is a diagram showing a display example in a case where the result of skin disease analysis by the skin disease analysis system 1 is a malignant tumor.
Fig. 7 is a diagram showing a display example in a case where the result of skin disease analysis by the skin disease analysis system 1 is a benign tumor.
Fig. 8 is a table showing an example of determination results by the skin disease analysis system 1 of the present embodiment.
Fig. 9 is a table showing an example of determination results by the skin disease analysis system 1 of the present embodiment.
Fig. 10 is a table showing an example of determination results by the skin disease analysis system 1 of the present embodiment.
Fig. 11 is a table showing an example of determination results by the skin disease analysis system 1 of the present embodiment.
Fig. 12 is a table showing an example of determination results by the skin disease analysis system 1 of the present embodiment.
Fig. 13 is a table showing an example of determination results by the skin disease analysis system 1 of the present embodiment.
Fig. 14 is a table showing an example of determination results by the skin disease analysis system 1 of the present embodiment.
Fig. 15 is a table showing an example of determination results by the skin disease analysis system 1 of the present embodiment.
Fig. 16 is a table showing an example of determination results by the skin disease analysis system 1 of the present embodiment.
Fig. 17 is a table showing an example of determination results by the skin disease analysis system 1 of the present embodiment.
Fig. 18 is a table showing an example of determination results by the skin disease analysis system 1 of the present embodiment.
Fig. 19 is a table showing an example of determination results by the skin disease analysis system 1 of the present embodiment.
Fig. 20 is a table showing an example of determination results by the skin disease analysis system 1 of the present embodiment.
Fig. 21 is a table showing three cases that have multiple image data for the case diagnosed with malignant melanoma and have different determination results (as the first candidate of AI determination).
Fig. 22 is a table showing a relationship between the confidence and the accuracy.
Fig. 23 is a table showing cases in which multiple images were determined for malignant melanoma (MM) and nevocellular nevus (NCN).
Fig. 24 is examples showing learning curves of the models used in the present invention.
Fig. 25 is examples showing learning curves of the models used in the present invention.
Fig. 26 is a table showing the accuracies and standard deviations in a case where the test data and the learning data are determined by learned models that have learned from the models used in the present invention.
Fig. 27 is a table showing a confusion matrix in a case where the test data is determined by using a learned model that has learned from DenseNet201.
Fig. 28 is a flowchart showing the process of re-prediction executed in the skin disease analyzer 200.
Fig. 29 is a table showing an example of determination results by the skin disease analysis system 1 of the present embodiment.
Fig. 30 is a table showing an example of determination results by the skin disease analysis system 1 of the present embodiment.
Fig. 31 is a table showing an example of determination results by the skin disease analysis system (equipped with three classifiers of a skin disease determination engine, a first-learned model, and a second-learned model) of the present embodiment.

### Description of Embodiments

The present invention is to provide a system in which an input image is analyzed, for example, by machine learning using a deep neural network, and a different type of skin tumor is determined from the image.

In the present invention, the image to be input is not limited only to an image of skin tumor, and the present invention can be applied, for example, even in a case where the quality of the image is poor, an image of a disease other than skin tumor such as inflammatory disease is input, or an image having nothing to do with skin tumor is erroneously input, or the like.

For example, skin cancer is caused mainly by UV rays in sunlight, and is more common in whites, less common in yellow races, and extremely rare in blacks. For example, the incidence of malignant melanoma is 1500 to 2000 people per year in Japan, but in the United States, the incidence is estimated to be around 91000 people per year.

In Japan, since the number of cases is small, the awareness of the disease is low, and further, it is often difficult to distinguish between malignant melanoma and lentigo. For example, in a case where the affected part does not show a black tone, the patient himself or herself makes a layperson's judgment that the affected part is acne or boil, and may not treat it (not go to hospital). Further, even in a case where the patient has gone to hospital, an accurate diagnosis may not be made when the patient goes to see a doctor other than a dermatologist. As a result, the disease may progress and the prognosis may worsen.

There are many malignant tumors other than malignant melanoma, and it is difficult to accurately distinguish among the malignant tumors unless the doctor is a skilled dermatologist.

However, a skin tumor has symptoms on the skin surface, and some changes can be recognized by the patient or anyone else. For this reason, if such a pathological condition can be easily determined at the time of being noticed, a skin tumor can be found in an early stage, and can be expected to be completely cured. In view of this, the present invention is to provide a skin disease analysis program, a skin disease analysis method, a skin disease analyzer, and a skin disease analysis system, which can be easily handled even by a primary care doctor such as a general internist, or a general practitioner, and can support the diagnosis of skin tumor.

Skin disease includes a disease showing a solitary lesion as in melanoma, and a disease showing a lesion spreading in a planar manner as in atopic dermatitis. Among them, a skin tumor is generally a solitary, but in some cases, a skin tumor showing multiple lesions as in mycosis fungoides (MF) or a skin tumor spreading in a planar manner is rarely observed. In addition, an inflammatory skin disease is generally a planar, but in some cases, an inflammatory skin disease showing a solitary is observed.

In the present specification, the term "skin tumor" refers to a skin disease showing a solitary lesion (including a skin disease in which individual lesions are surrounded by normal skin and can be recognized independently from other lesions, even if the skin disease shows multiple lesions). Specific examples of the "skin tumor" include tumors of 24-class shown below. Examples of the malignant tumor include actinic keratosis (AK), actinic keratosis with cutaneous horn (AKhorn), Bowen's disease (Bowen), squamous cell carcinoma (SCC), basal cell carcinoma (BCC), amelanotic basal cell carcinoma (BCCamela), extramammary Paget's disease (EMPD), malignant melanoma (MM), amelanotic malignant melanoma (MMamela), and angiosarcoma (AS). Further, examples of the benign tumor include poroma (Poroma), amelanotic poroma (Poromamela), sebaceous nevus (SebaceousN), seborrheic keratosis (SK), amelanotic seborrheic keratosis (SKamela), blue nevus (BlueN), congenital melanocytic nevus (CongenitalN), nevocellular nevus (NCN), amelanotic nevocellular nevus (NCNamela), spitz nevus (Spitz), amelanotic spitz nevus (Spitzamela), lentigo (Lentigo), nevus spilus (Spilus), and pyogenic granuloma (PG).

In this regard, mycosis fungoides (MF) is a hematological tumor, although it is classified as a skin tumor, and does not necessarily form any neoplastic lesions, or may show multiple lesions, and thus mycosis fungoides (MF) is not included in the skin tumor in the present specification.

In the present invention, as the camera that takes a photograph of an affected part of skin tumor, any camera can be used regardless of the model as long as the camera has a resolution above a certain level. In view of the convenience of being able to input the photograph of an affected part as it is or after processing it, into a skin disease analyzer even from a remote place, it is preferable to use a smartphone or tablet computer with a built-in camera.

Further, in order to improve the accuracy of determination in the skin disease analyzer, it is preferable that the photographic image of an affected part does not include any part other than the skin. For this reason, in the present invention, it is preferable to use an application that crops the image so that the lesion is put almost in the central part without including any part other than the skin, before inputting the photographic image into the skin disease analyzer. This application for cropping is a program for fitting an image of an affected part into a predetermined format in order to facilitate the analysis of the image by a skin disease determination engine and a learned model.

### <Hardware configuration of skin disease analysis system 1>

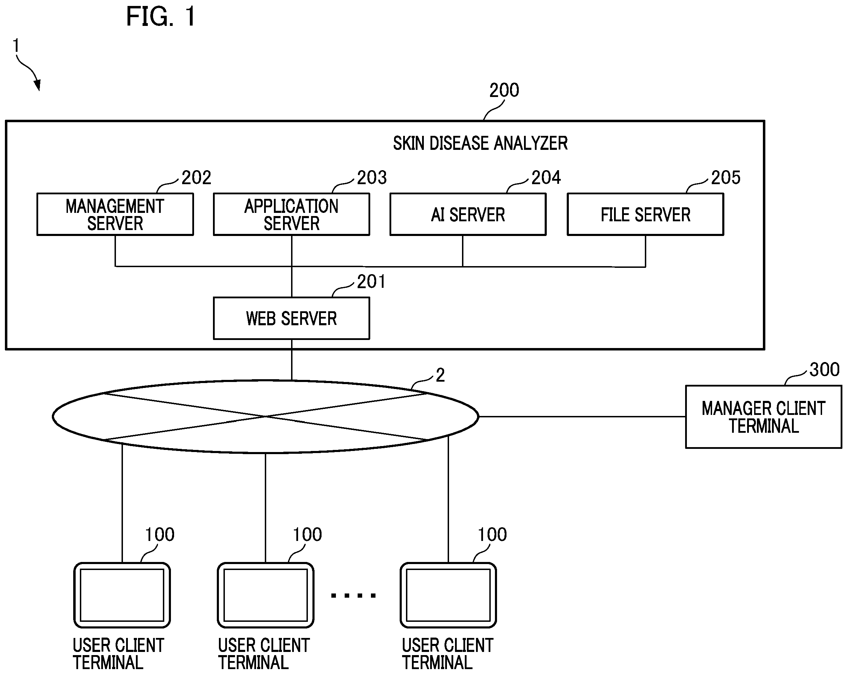

Fig. 1 is a block diagram showing a hardware configuration of the skin disease analysis system 1 according to one embodiment of the present invention. As shown in the diagram, the skin disease analysis system 1 includes one or more of user client terminals 100, a skin disease analyzer 200 that is a skin disease analysis server, and a manager client terminal 300. The user client terminals 100 and the manager client terminal 300 are connected to the skin disease analyzer 200 via a network 2. The network 2 is a wired or wireless communication system such as the Internet. The skin disease analysis program itself according to the present embodiment, and the data required to execute the program are stored in storage devices of the user client terminals 100, the skin disease analyzer 200, and the manager client terminal 300. The storage device of the skin disease analyzer 200 may be, for example, a file server 205.

The skin disease analyzer 200 may be any computer such as a personal computer, a workstation, or a general-purpose computer, or may also be any combination thereof. The skin disease analyzer 200 may be one computer, or may also be multiple computers.

The skin disease analyzer 200 includes a web server 201, a management server 202, an application server 203, an AI server 204, and a file server 205. Each server of the skin disease analyzer 200 functions as a control unit that controls the operation of the skin disease analyzer 200 by executing the skin disease analysis program.

The web server 201 communicates via the network 2. The management server 202 performs the account management of the skin disease analysis system 1. The application server 203 provides various applications such as a user interface with a user who operates the user client terminal 100 or the manager client terminal 300, and cropping of image data. The AI server 204 executes skin disease analysis on an image input from the user client terminal 100. The file server 205 stores and manages various data. The web server 201, the management server 202, the application server 203, the AI server 204, and the file server 205 are connected to one another, and transmit and receive various data.

The file server 205 of the skin disease analyzer 200 stores facility information that is information about medical facilities, account information that is information about accounts of operators (such as a doctor, a medical-care worker, a patient, and a system manager) of the user client terminals 100, various data input from the user client terminals 100, programs and data that are required for the operation of the skin disease analysis system 1, and the like.

The user client terminal 100 may be a mobile terminal such as a smartphone, a feature phone, a PDA (personal digital assistant), or a tablet computer, or may also be a desktop computer or the like. It is desirable that the user client terminal 100 can be operated with a touch panel. It is desirable that the user client terminal 100 is operated by a doctor, but the user client terminal 100 may be operated by a medical-care worker other than a doctor, or a patient himself or herself.

The user client terminal 100 executes account management of an operator (such as a doctor, a subject to be inspected, or a health service manager) of the user client terminal 100, taking a photograph of an affected part, transmission of the photographed image to the skin disease analyzer 200, display of various reports such as analysis results by the skin disease analyzer 200, and the like. The photograph of an affected part is taken by an imaging device such as a camera other than the camera of the user client terminals 100, and then the image data photographed by the imaging device may be input into the user client terminals 100.

The manager client terminal 300 may be a mobile terminal such as a smartphone, a feature phone, a PDA (personal digital assistant), or a tablet computer, or may also be a desktop computer or the like. The manager client terminal 300 may have the same configuration as or a different configuration from that of the user client terminal 100.

The manager client terminal 300 executes account management of an operator (such as a system manager) of the manager client terminal 300, display of various reports such as operational status of the skin disease analysis system 1, and the like.

### <Functional configuration of AI server 204>

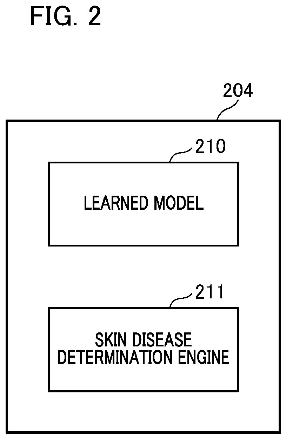

Fig. 2 is a block diagram showing a functional configuration of the AI server 204 shown in Fig. 1.

The AI server 204 includes a learned Model 210, and a skin disease determination engine 211. The learned Model 210 is a learned model for predicting the kind of skin disease, which has machine learned from images of affected parts of various skin diseases in advance. The skin disease determination engine 211 determines whether or not the disease is a skin disease, or determines whether or not the disease is a skin tumor. As will be described in detail later, the AI server 204 performs skin disease analysis on the image data transmitted from the user client terminal 100.

### <Explanation of operation of skin disease analysis system 1>

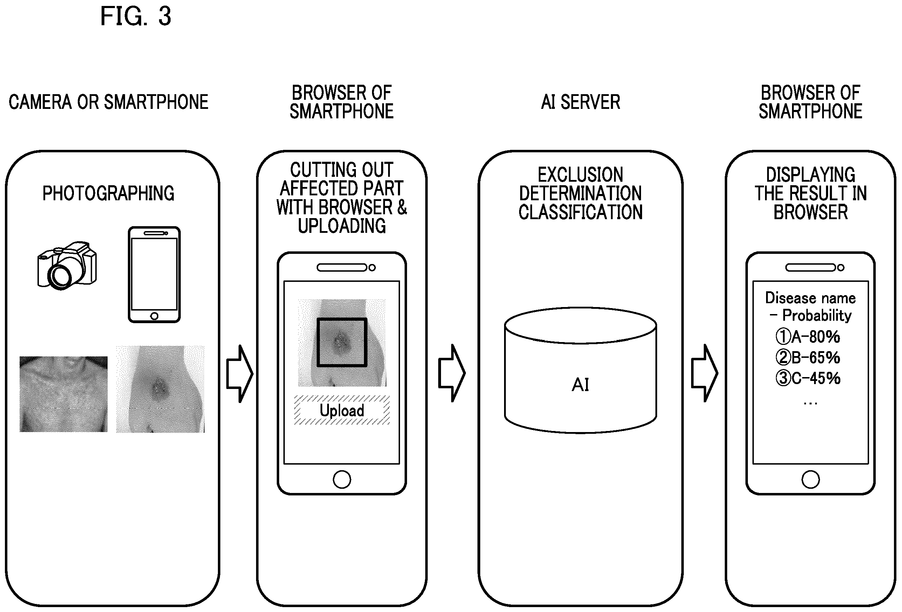

Fig. 3 is a diagram illustrating an outline of operation of the skin disease analysis system 1 shown in Fig. 1. Fig. 3 shows an example in a case where the user client terminal 100 is a smartphone.

First, a photograph of an affected part is taken by a camera separated from the user client terminal 100, or a camera built in the user client terminal 100. In a case where the photograph is taken by a separated camera, the photographed image data may be sent to the user client terminal 100 via a detachable storage medium, or may be sent to the user client terminal 100 by communication.

After that, cropping is performed so that any part other than the skin is excluded from the photographed image data. This cropping may be performed by an application program executed by the user client terminal 100. Further, in the user client terminal 100, a browser (web browser) is executed to connect the user client terminal 100 to the web server 201 of the skin disease analyzer 200, and the cropping may be performed with an application program executed by the application server 203 of the skin disease analyzer 200. It is preferable that the application for executing the cropping crops an image so that the lesion is put almost in the central part without including any part other than the skin.

Subsequently, the user client terminal 100 uploads the cropped image data to the skin disease analyzer 200. In a case where the cropping of image data is performed on the skin disease analyzer 200 side, the user client terminal 100 uploads the image data before cropping to the skin disease analyzer 200.

As to the image to be uploaded, one or more images are sufficient for each case, but in order to avoid the retaking if the image is blurry, or since there is a possibility of erroneous determination depending on the image states such as an angle for taking a photograph, distance, and brightness, it is preferable to upload two or more images and use them for the determination. When there are extremely many images to be uploaded, it will be complicated, and thus it is preferable to upload from 2 to 10 images, and particularly preferable to upload from 3 to 5 images.

In the skin disease analyzer 200 to which image data have uploaded, exclusion determination and classification are performed by the AI server 204, as will be described in detail later. The results of the analysis by the AI server 204 are transmitted to the user client terminal 100 via the web server 201, and the user client terminal 100 that has received the results displays the analysis results with the browser. At this time, for example, the disease name and the probability (also referred to as "confidence") of the disease are displayed up to the third candidate in descending order of the probability. Alternatively, it is also possible to simply display whether the disease is malignant or benign without displaying the disease name. Further, at this time, the coping method of the disease may be displayed. As the coping method, follow-up, excision of the affected part, and the like are included.

### <Explanation of processing flow of skin disease analysis system 1>

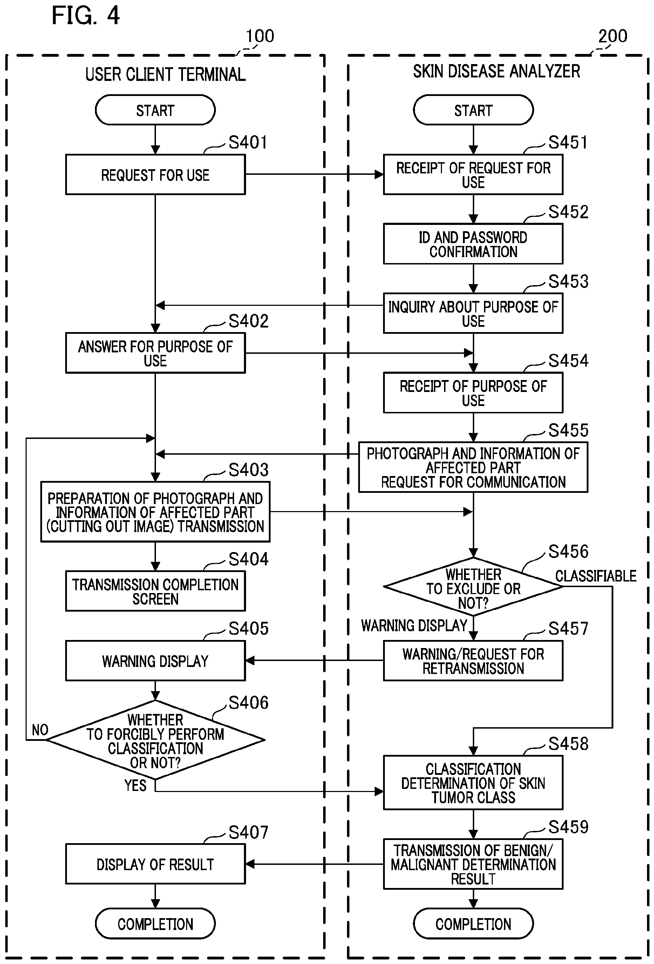

Fig. 4 is a flowchart showing an example of the process of the skin disease analysis program executed in the skin disease analysis system 1. In this case, the process of analyzing the skin disease is executed by the user client terminal 100 and the skin disease analyzer 200.

First, the user client terminal 100 is connected to the skin disease analyzer 200 in response to the operation of the user, and transmits a request for use of the skin disease analysis system 1 to the skin disease analyzer 200 (Step S401). The skin disease analyzer 200 receives the request for use from the user client terminal 100 (Step S451). The request for use includes an ID registered in advance for the user of the user client terminal 100 and a password associated with the ID. The ID and password are registered and stored in advance, for example, in the file server 205.

The management server 202 collates the ID and password stored in the file server 205 with the ID and password included in the request for use from the user client terminal 100, and confirms whether or not the user who has sent the request for use is the valid user (Step S452).

In the skin disease analyzer 200, in a case where the user who has sent the request for use is determined not to be the valid user in Step S452, the skin disease analyzer 200 transmits a message to that effect to the user client terminal 100 and rejects the use.

In a case where the user who has sent the request for use is determined to be the valid user in Step S452, the skin disease analyzer 200 inquires in the user client terminal 100 about the purpose of use (Step S453). The inquiry about the purpose of use may request the input of, for example, "diagnostic purpose", "research purpose", or the like from the user.

The user who operates the user client terminal 100 transmits an answer for the purpose of use to the skin disease analyzer 200 (Step S402), and the skin disease analyzer 200 receives the purpose of use (Step S454).

Next, the skin disease analyzer 200 transmits to the user client terminal 100 the request for transmission of a photograph and information of an affected part (Step S455). Upon receiving the request for transmission of a photograph and information of the affected part, the user client terminal 100 takes a photograph of the affected part and transmits the image data to the skin disease analyzer 200 (Step S403). The user client terminal 100 transmits the photograph of the affected part in Step S403, and then displays a transmission completion screen to the user (Step S404). In this regard, the photograph of the affected part may be taken before the request for use in Step S401. In Fig. 4, the information of an affected part described in Step S455 and Step S403 includes the image data obtained by performing the above cropping on the image data of the photograph of an affected part. As described above, the cropping may be performed in the user client terminal 100 or the skin disease analyzer 200. In a case where the cropping is performed in the skin disease analyzer 200, the information of an affected part does not include the cropped image data.

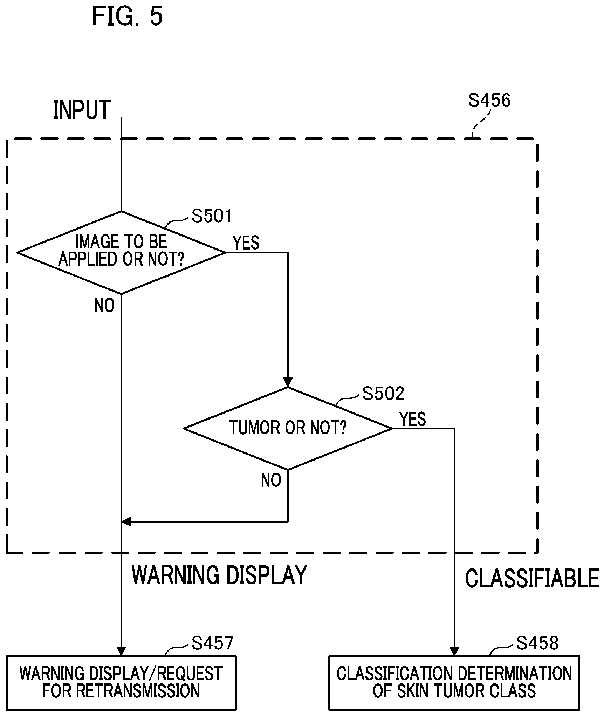

In a preferred embodiment of the present invention, the skin disease analyzer 200 makes an exclusion determination for the received photograph of an affected part (S456). This exclusion determination will be described with reference to Fig. 5.

Fig. 5 is a flowchart showing the process of exclusion determination executed in the skin disease analyzer 200. The skin disease analyzer 200 determines whether the received image data of a photograph of an affected part is an image to be applied or an image to be excluded (Step S501). In this case, for example, a skin image can be taken as the image to be applied. It may be determined whether or not the image is a skin image by learning skin images in the past. In a case of an image to be excluded, the process proceeds to Step S457 as a "warning display". In a case of an image to be applied, the process proceeds to Step S502. In Step S502, the skin disease analyzer 200 determines whether or not the image data is a tumor image. In a case of an image other than tumor images such as an inflammatory image, the process proceeds to Step S457 as a "warning display". In a case of a tumor image, the process proceeds to Step S458 as "classifiable". The process of the exclusion determination will be described in detail later.

Returning to the explanation of Fig. 4, in a case where the "warning display" has been selected in the exclusion determination of Step S456, the skin disease analyzer 200 transmits warning/request for retransmission to the user client terminal 100 (S457). This "warning display" means that the photograph of an affected part received from the user client terminal 100 is determined not to be a tumor image to be applied in the skin disease analysis system 1, and in Step S457, a warning is issued to the user client terminal 100, and further retransmission of a correct photograph of the affected part is requested. In response to this, a warning display is output to the user in the user client terminal 100 (Step S405). In response to this warning display, the user selects whether to retransmit the image data by, for example, taking a photograph of the affected part again, or to forcibly execute the skin disease analysis (classification) of the image data with the warning display, and the selected instruction is input to the user client terminal 100.

In a case where the user has selected the retransmission of image data, returning to Step S403, the user client terminal 100 retransmits image data of a photograph of the affected part to the skin disease analyzer 200. In a case where the user has selected the forced execution of the skin disease analysis (classification) of the image data with the warning display, the instruction of forced execution of the skin disease analysis (classification) is transmitted to the skin disease analyzer 200 (Step S406). In a case where the instruction to forcibly determine the classification has been received, the skin disease analyzer 200 proceeds to Step S458 and continues the process.

In a case of a system in which multiple images of an affected part can be transmitted from the user client terminal 100, exclusion determination of Step S456 is performed for each of the transmitted images, and for example, when all of the images are determined "not to be a tumor image", warning/request for retransmission is transmitted to the user client terminal 100 (S457).

Further, in a system in which three or more images of an affected part can be transmitted from the user client terminal 100, it can be appropriately set whether or not the warning/request for retransmission (S457) is transmitted to the user client terminal 100 in a case where several images are determined "not to be a tumor image" in Step S456.

The skin disease analyzer 200 determines the classification of skin tumors in Step S458. The classification will be described in detail later. The determination results of Step S458 include a determination result of benign tumor or malignant tumor, a disease name, and a probability of being a disease of the disease name. In Step S459, the skin disease analyzer 200 transmits the determination results of Step S458 to the user client terminal 100 and ends the process.

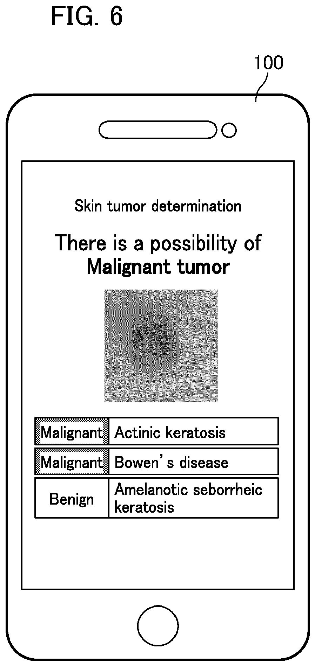

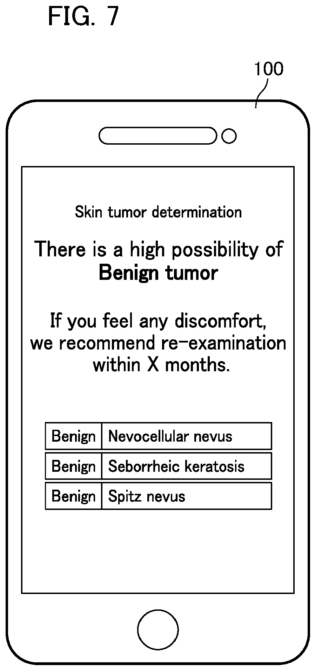

The user client terminal 100 that has received the determination results of Step S458 displays the determination results to the user (S407), and ends the process. Display examples of Step S407 are shown in Figs. 6 and 7. Fig. 6 is a diagram showing a display example in a case where the result of skin disease analysis by the skin disease analysis system 1 is a malignant tumor. Fig. 7 is a diagram showing a display example in a case where the result of skin disease analysis by the skin disease analysis system 1 is a benign tumor.

As a result of the determination, in the example of Fig. 6, it is indicated that both of a disease having the highest probability and a disease having the next highest probability are malignant tumors, and indicated that there is a possibility of malignant tumor. As a result of the determination, in the example of Fig. 7, it is indicated that diseases with up to the third highest probability are benign tumors, and that there is a high possibility of benign tumor.

In addition, the history of determination results in Step S458 may be stored in the file server 205 so as to be viewed by a user who operates the user client terminal 100 or an operator of the manager client terminal 300. Further, the history of determination results may be managed by associating with a user who operates the user client terminal 100 or with the patient of the determined image.

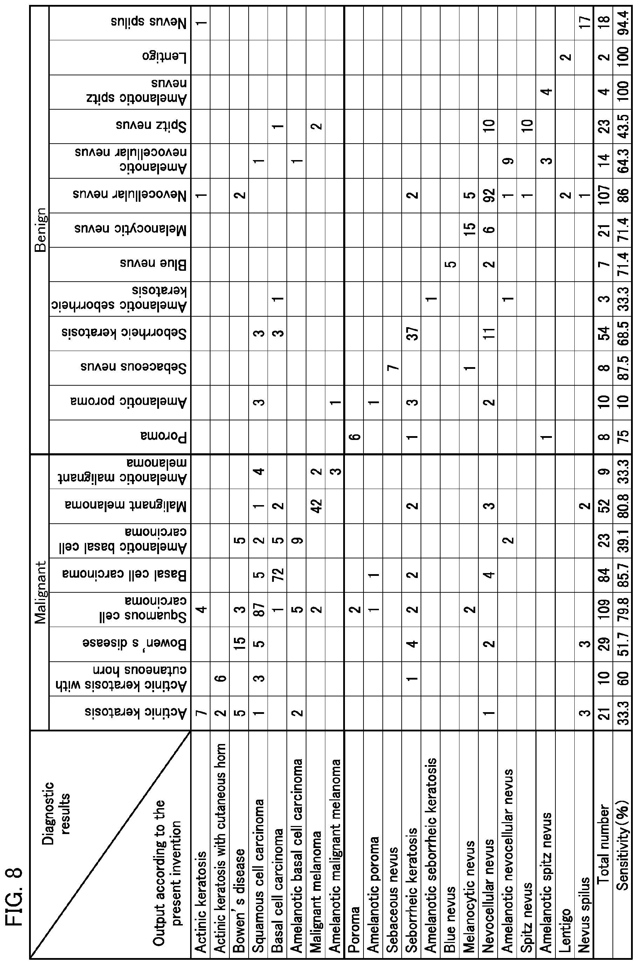

Fig. 8 is a table showing an example of determination results by the skin disease analysis system 1 of the present embodiment. In Fig. 8, the diagnostic results by a specialist are listed in the horizontal direction, and the results (output according to the present invention) obtained by determining the diagnosed image of an affected part by the skin disease analysis system 1 are listed in the vertical direction. For example, in each of the columns on the leftmost with disease names lined up side by side in the horizontal direction, the number of images from patients diagnosed with "actinic keratosis" is shown, and the total number is 21. In the determination results by the skin disease analysis system 1, among these 21 cases, 7 cases have been determined to be "actinic keratosis", 2 cases have been determined to be "actinic keratosis with cutaneous horn", 5 cases have been determined to be "Bowen's disease", 1 case has been determined to be "squamous cell carcinoma", 2 cases have been determined to be "amelanotic basal cell carcinoma", 1 case has been determined to be "nevocellular nevus", and 3 cases have been determined to be "nevus spilus". According to the determination results, among the 21 cases of actinic keratosis being malignant tumor, 17 cases have been determined to be malignant tumor, 4 cases have been determined to be benign tumor, and a sufficient sensitivity of 81% has been obtained as the sensitivity for malignant tumor. As shown in Fig. 8 and Figs. 9 and 29 to be described later, the malignant tumors tend to be mistaken for each other, and the benign tumors also tend to be mistaken for each other. This indicates that the malignant tumors are similar to each other, and the benign tumors are also similar to each other, in the image. From these results, it becomes possible to predict whether the tumor is a malignant tumor or a benign tumor by using the determination results of large number-class (for example, 14 to 24-class).

### <Explanation of operation of AI server 204>

The program executed by the AI server 204 of the present embodiment includes a first step (Step S456 in Fig. 4) of determining to exclude an image other than skin tumor images from image data (images to be analyzed), and a second step (Step S458 in Fig. 4) of determining which kind of skin tumor the image, which has not been excluded in the first step as being a skin tumor image, indicates. In the first step, for example, the skin disease determination engine 211 determines whether or not the image data of an affected part indicates a skin tumor image. In the second step, for example, the skin disease determination engine 211 predicts the kind of skin tumor of the image data of an affected part, which has been determined to be a skin tumor image by the first step, by a learned Model 210.

### <First step>

The first step is aimed at narrowing the input images down to images of skin tumors. A classifier used in the second step determines whether the tumor is a benign tumor or a malignant tumor in 2-class, determines which one of a malignant tumor of epitheliocytes, a malignant tumor of melanocytes, a benign tumor of epitheliocytes, and a benign tumor of melanocytes in the above-mentioned 4-class, determines which one of the tumors of 4-class, a malignant tumor of vascular component, and a benign tumor of vascular component in 6-class, or determines the kind of skin tumor in 14 to 24 or more-class, which has been further finely classified, with the proviso that it is premised that the image to be input in the classifier is an image of skin tumor. Accordingly, even in a case where any image other than images of skin tumors has been input, the image is determined whether it indicates benign or malignant, and it is determined which one of the kinds of the 4 to 24-class the image is. That is, for example, even if the image is an image of inflammatory skin disease, the image is determined to be a skin tumor, and even in a case where an image that has not shown the original tumor lesion because the image is blurry or where an image that has nothing to do with the skin is erroneously input, the image is determined to be a skin tumor, which may bring about false recognition to the user. In view of this, in the present embodiment, it is preferable to provide a first step that is a step of excluding any image other than images of skin diseases.

This first step (sometimes also referred to as "exclusion determination step") system is not particularly limited as long as images to be input in the second step can be narrowed down to images of skin tumors, and examples of the system include:
(1) a system of determining whether or not the image is a skin tumor image in one stage;
(2) a system of excluding any image other than images of the skin at first, and determining whether or not each of the remaining skin images is a skin tumor image, in two stages;
(3) a system of excluding any image other than images of skin diseases at first, and determining whether or not each of the remaining skin disease images is a skin tumor image (excluding an inflammatory skin disease image), in two stages; and
(4) a system of excluding any image that shows something other than the skin at first by the distribution of colors, and determining whether or not each of the remaining images is a skin tumor image, in two stages.

In a case of being excluded by the first step, a message that the transmitted image has been excluded because of having a possibility of not being an image of skin tumor is transmitted to the user, (corresponding to Step S405). Specific examples of the message, which requires confirmation of the user, include "Detected that the transmitted image may not be a skin tumor image. Sorry to trouble you, but please reconfirm the transmitted image. After confirmation, please retransmit the same image or transmit the re-taken one." and the like

### <Second step>

The second step is a step of determining the type of skin tumor by a classifier. As an example, a system of classifying skin tumors into 24 classes will be specifically described.

Herein, in the 24-class of skin tumors, malignant tumors are actinic keratosis (AK), actinic keratosis with cutaneous horn (AKhorn), Bowen's disease (Bowen), squamous cell carcinoma (SCC), basal cell carcinoma (BCC), amelanotic basal cell carcinoma (BCCamela), extramammary Paget's disease (EMPD), malignant melanoma (MM), amelanotic malignant melanoma (MMamela), and angiosarcoma (AS); and benign tumors are poroma (Poroma), amelanotic poroma (Poromamela), sebaceous nevus (SebaceousN), seborrheic keratosis (SK), amelanotic seborrheic keratosis (SKamela), blue nevus (BlueN), congenital melanocytic nevus (CongenitalN), nevocellular nevus (NCN), amelanotic nevocellular nevus (NCNamela), spitz nevus (Spitz), amelanotic spitz nevus (Spitzamela), lentigo (Lentigo), nevus spilus (Spilus), and pyogenic granuloma (PG).

In this regard, in the present invention, a tumor other than the tumors of the 24-class can also be determined by a classifier that has allowed a model to learn from as many various tumor images available as possible as the learning data. Further, even if all of the 24-class are not learned, for example, it is possible to determine by a classifier that has learned by selecting a tumor class having many cases. Accordingly, the kinds (classes) of tumors presumed in the second step are usually 2 to 100-class, preferably 4 to 50-class, and furthermore preferably 10 to 30-class.

As the classifier, for example, a learned model that has machine learned from skin tumor images obtained by taking photographs of affected parts of diseases in a model in which the initial value of weight has been determined from ImageNet data is used. Specific examples of the model used in the present invention include ResNet50, DenseNet169, DenseNet201, InceptionResNetV2, VGG16, VGG19, MobileNet, DenseNet121, Xeption, and InceptionV3, but are not limited thereto.

When an image of skin tumor to be determined is input to this classifier, the rank of certainty of the skin tumor determined by the classifier, and the probability thereof are generally output. The output value can be converted to an appropriate numerical value, words, or the like depending on the purpose, and displayed for the user.

For example, since a primary care doctor (a general practitioner or other doctors, whom a person who thinks of having some kind of disease usually consults first) is often not a dermatologist, it is assumed that it may be difficult to determine the kind of skin tumor. However, in a case of malignant tumor, if the malignant tumor is left untreated, it may progress and metastasize to other places, and the prognosis may worsen. Accordingly, as the output display for a primary care doctor, determination of benign or malignant is displayed, and in a case where the classifier has predicted to be malignant, a message prompting a response such as a referral to a dermatologist is issued. In this way, even if the tumor is a malignant skin tumor, the malignant skin tumor can be treated without metastasis if it can be found in an early stage. Further, the existence of skin tumor is relatively easy to understand for both a patient himself or herself and others, and thus it is conceivable that the patient feels free to consult with a primary care doctor about the skin tumor. At this time, if the primary care doctor can determine the skin tumor by utilizing the skin disease analysis system 1 of the present embodiment as a user, it becomes possible to give a referral to a dermatologist as needed, and to address it as soon as possible. In this case, the user client terminal 100 may display only the benign or malignant to the user as the determination result in Step (S407).

In addition, it is considered that it is necessary for a dermatologist to determine the type of skin tumor and decide the therapeutic strategy according to the type. In view of this, in a case where a dermatologist uses the skin disease analysis system 1 of the present embodiment as a user, for example, it can be mentioned that the classifier displays the types of the skin tumor in the top 3 to top 5 determined to be highly probable.

The skin disease analysis system 1 according to the present embodiment can also perform an operation in which the display content in Step (S407) is made different depending on a user type, for example, by managing the user type, that is, whether the user is a primary care doctor (doctor other than a dermatologist) or a dermatologist, and displaying a message for such a type of the user, in the management server 202.

With reference to the highly probable kind of skin tumor output by the skin disease analysis system 1 of the present embodiment, the dermatologist grasps various parameters that are difficult to measure with a plane image, such as a surface shape of a tumor, unevenness, a size, patient age, and an odor of an affected part, and makes a comprehensive determination to diagnose as a skin tumor.

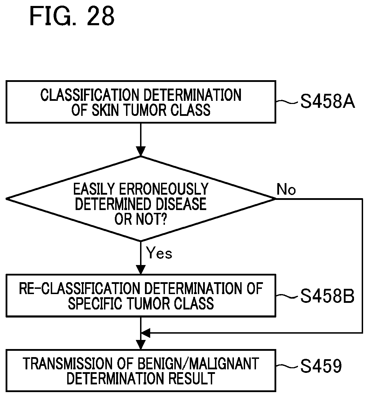

In this regard, the AI server 204 includes a skin disease re-prediction engine, and the skin disease analysis program may allow the computer to execute a third step of re-prediction by the skin disease re-prediction engine. In this third step, by the skin disease re-prediction engine (second-learned model), the kind of skin disease is re-predicted from the information of an affected part and the kind of skin tumor predicted in the second step. This re-prediction will be described with reference to Fig. 28.

Fig. 28 shows that re-determination is performed (S458B) by using a classifier (second-learned model) that has been created by machine learning from images of affected parts of specific skin diseases including skin tumors that are easily erroneously determined, in a case where a disease has been classified in a disease class in which the disease is easily mistaken for other diseases as a result of performing the classification determination of skin tumor class (S458A). For example, since the classifier (first-learned model) that performs 24-class classification learns from images of various kinds of diseases, the classifier may erroneously determine diseases having similar shapes to each other. In this regard, a classifier (re-determination engine of specifically, for example, 4-tumor class of basal cell carcinoma, malignant melanoma, seborrheic keratosis, and nevocellular nevus) that has been created by learning only from images of a small number of specific diseases can more accurately classify diseases that are easily mistaken for each other.

At this time, a learned model for re-predicting the kind of skin disease from the information of an affected part and the kind of skin tumor may be used. The information of an affected part includes one or multiple of patient age, a size of an affected part, an odor of an affected part, a site of disease development, and a three-dimensional shape of an affected part. This information of an affected part corresponds to the information of an affected part described in Step S455 and Step S403 of Fig. 4, and can be transmitted to the skin disease analyzer 200 by the user. The result of the re-prediction by the third step can be displayed on the user client terminal 100 similarly to the result of the prediction by the second step.

As the configuration of the classifier in the second step, a skin tumor of 14 to 24-class can be determined in one stage, and further a step of two or more stages may be included. In a case where the determination is performed in two stages, for example,
(1) a method in which a tumor is separated into a benign tumor group and a malignant tumor group in the first stage (Step A), and the benign tumor group and the malignant tumor group are each determined to be any of disease classes in the second stage (Step B),
(2) a method in which a tumor is separated into a melanotic tumors and an amelanotic tumors in Step A, and the melanotic tumors and the amelanotic tumors are each determined to be any of disease classes in Step B,
(3) a method in which diseases are classified into diseases of 4-class, 14-class, 17-class, and the like in Step A, and the classified diseases are each determined to be any of classes in Step B, or the like can be used.

Further, some tumors have morphologies similar to each other in the image, and there are combinations with which a classifier (learned model) is easy to make a mistake (erroneous determination) in the diagnostic imaging. In view of this, it can be expected to improve the accuracy by, for example, a method in which diseases are determined to be any of disease classes (14-class, 17-class, 21-class, 24-class, and the like) in the above second step, and then a specific disease that is easily erroneously determined is re-determined as the third step.

That is, the classifier used in the third step is a classifier that has learned by selecting some specific disease classes from the disease classes that have been learned by the classifier used in the second step. Therefore, the number of disease classes learned by the classifier used in the third step is smaller than the number of disease classes used in the second step.

Further, herein, the combination of disease classes that are easily mistaken for each other is in a case where roughly 2% or more of the case images of specific diseases definitely diagnosed by a specialist are determined to be other specific diseases by a classifier, or in a case where roughly 2% or more of the case images of specific diseases determined by a classifier of a certain disease are diagnosed as other specific diseases by an actual specialist, and means a combination in which these cases are recognized as each other in two or more disease classes.

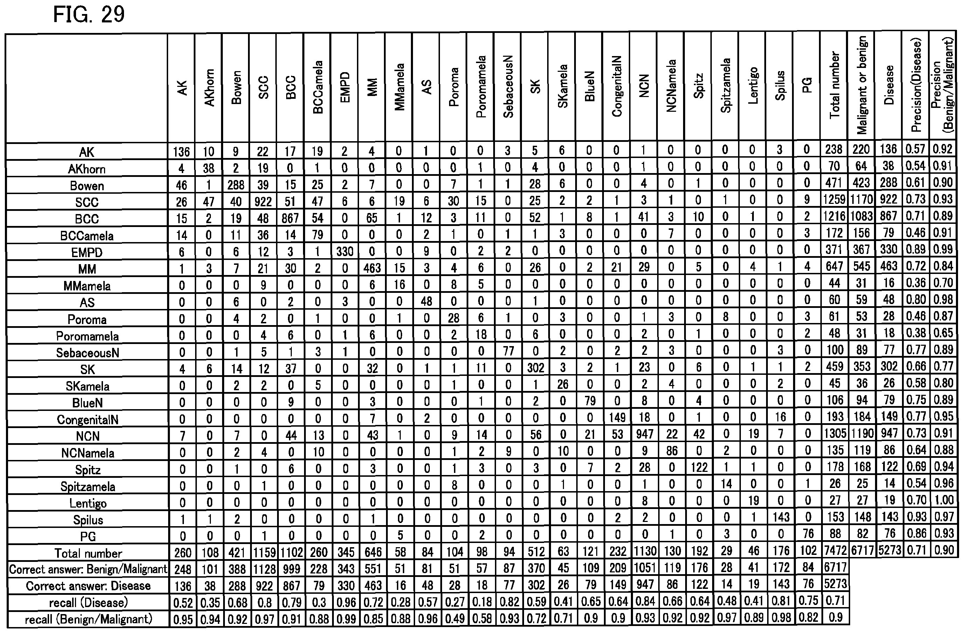

Specifically, in Fig. 29, the number of images of MM is 646 as the actual diagnostic result, and 43 images (6.66%) among the 646 images are determined to be NCN by a classifier (24-class determination device). In addition, the number of the images determined to be NCN by the classifier (24-class determination device) is 1305, but the number of the images actually diagnosed as MM is 43 (3.30%) among the 1305 images, and thus, MM and NCN are a combination of disease classes that are easily mistaken for each other. Similarly to this, in the classifier that has given the determination results shown in Fig. 29, in addition to MM (malignant) and NCN (benign), SK (benign) and BCC (malignant) are also a combination of disease classes that are easily mistaken for each other.

Further, the number of the images of Poromamela actually diagnosed is 98, but 11 images (11.22%) among the 98 images are determined to be BCC by the classifier (24-class determination device). In addition, the number of the images of Poromamela determined by the classifier is 48, but the number of the images actually diagnosed as BCC is 6 (12.50%) among the 48 images, and thus, BCC and Poromamela are a combination of disease classes that are easily mistaken for each other.

Further, in addition to these combinations, for example, Bowen (malignant) and SCC (malignant), SCC (malignant) and BCC (malignant), NCN (benign) and Spitz (benign), NCN (benign) and Lentigo (benign), or the like is also a combination of disease classes that are easily mistaken for each other. In this regard, in a case of determining whether it is malignant or benign, for example, for the purpose of screening for a malignant tumor, and in a case where a certain benign tumor and a certain malignant tumor are a combination of disease classes that are easily mistaken for each other, it is particularly important because such cases cause false negatives or false positives.

As described above, since malignant melanoma (MM) and nevocellular nevus (NCN) are extremely similar to each other in shape, they may be erroneously determined by a classifier. In view of this, in a case where the image has been determined to be malignant melanoma or nevocellular nevus in the second step, in order to accurately distinguish these two kinds, the image is determined to be either malignant melanoma or nevocellular nevus in the third step by using a model that has learned only from images of malignant melanoma and nevocellular nevus.

Further, from the viewpoint of not overlooking any malignancy, in a case where the image has been determined to be nevocellular nevus in the second step, the image is determined to be either malignant melanoma or nevocellular nevus again by using a model that has learned only from images of malignant melanoma and nevocellular nevus, in the third step. In this case, in a case where the image has been determined to be malignant melanoma in the second step, the determination result of the second step is adopted as it is.

Furthermore, a tumor that is similar to malignant melanoma or nevocellular nevus may be added in the determination of the third step. Specifically, since it is considered that four kinds of malignant melanoma, basal cell carcinoma, seborrheic keratosis, and nevocellular nevus are similar to each other in shape, in a case where the image has been determined to be one of these four kinds in the second step, it is determined which one of these four kinds the image is again by using a model that has learned only from images of malignant melanoma, basal cell carcinoma, seborrheic keratosis, and nevocellular nevus, in the third step.

From the viewpoint of not overlooking any malignancy, in a case where the image has been determined to be seborrheic keratosis or nevocellular nevus in the second step, it is determined which one of these four kinds the image is again by using a model that has learned only from images of malignant melanoma, basal cell carcinoma, seborrheic keratosis, and nevocellular nevus, in the third step. In this case, in a case where the image has been determined to be malignant melanoma or basal cell carcinoma in the second step, the determination result of the second step is adopted as it is.

In addition, since it is considered that it varies depending on the learned model (first-learned model) used in the second step which tumors are similar to each other and are easily mistaken for each other as the classifier (AI), the kinds of tumor to be re-determined in the third step can be appropriately selected depending on the output content of the first-learned model used in the second step.

For example, in a case where the first-learned model makes a determination of 14 to 24-class, as the second-learned model used in the third step, a model that has learned by specifying the tumors of usually 2 to 10-class, preferably 3 to 8-class, and more preferably 4 to 6-class, which are selected from 14 to 24-class, is used.

In a preferred embodiment of the present invention, in a case where a tumor class determination step (second step) of predicting the kind of skin tumor has been performed, and in a case where the determination result of the tumor class determination step has been one kind of skin tumors that are easily mistaken for each other, a specific tumor class re-determination step (third step) of making a re-determination is executed by a second learned model that has machine learned from images of affected parts of specific diseases including the skin tumors that are easily mistaken for each other.

In a preferred embodiment of the present invention, in a case where a tumor class determination step (second step) of predicting the kind of skin tumor has been performed, and in a case where the determination result of the tumor class determination step has been one kind of benign tumors, and one kind of skin tumors that are easily erroneously determined to be the benign tumor has been a malignant tumor, a specific tumor class re-determination step (third step) of making a re-determination is executed by a second learned model that has machine learned from images of affected parts of specific diseases including the benign skin tumors and malignant skin tumors.

In a more preferred embodiment of the present invention, in a case where one kind of skin tumors that are easily mistaken for each other includes malignant melanoma, that is, in a case where a tumor class determination step (second step) of predicting the kind of skin tumor has been performed, and in a case where the analysis result of the tumor class determination step has been one kind of skin tumors that are easily erroneously determined to be malignant melanoma, a specific tumor class re-determination step (third step) of making a re-determination is executed by a second learned model that has machine learned from images of affected parts of diseases including malignant melanoma, and the skin tumors that are easily erroneously determined to be malignant melanoma.

Depending on the learned model, it may be effective to make a re-determination even in a case of a combination other than the combination including the above malignant melanoma. For example, a combination including amelanotic basal cell carcinoma, amelanotic seborrheic keratosis, and amelanotic nevocellular nevus is listed as the combination that easily causes erroneous determination.

Hereinafter, the skin disease analysis system 1 of the present embodiment will be described in more detail.

Preferred embodiment:
As the means for photographing an image of an affected part, it is preferable to use a portable device such as a smartphone with a camera or a tablet computer with a camera.

It is preferable that the smartphone used in the present embodiment is provided with an application for photographing an image of an affected part.

It is preferable that the application for photographing an image of an affected part has a function of arranging the tumor part in the central part in the screen and the normal skin part in the peripheral part in the screen, in the image of an affected part. By photographing an image in this way, it becomes possible to correctly recognize a skin tumor as the skin tumor.

The cropping of the image of an affected part may be performed by a doctor who is the user of the skin disease analysis system 1 while operating an application for cropping. In this case, the doctor visually recognizes the image of an affected part, and can operate the application so that the part other than the skin is excluded by arranging the tumor part in the central part and the normal skin part in the peripheral part, in the screen.

The patient himself or herself photographs the image of an affected part, and transmits the photographed image to a doctor, and then the doctor who is a user of the skin disease analysis system 1 may transmit the image data of the affected part to the skin disease analyzer 200 while operating the user client terminal 100. By adopting this system, it is possible to make a determination of a skin tumor even in a case where the patient is in a remote location.

Hereinafter, embodiments of the first step, second step, and third step described above will be mentioned. The present invention includes an aspect having a first step and a second step, an aspect having a second step and a third step, and an aspect having first to third steps. In this regard, an elimination determination device is used in the first step, a first-learned model is used in the second step, and a second-learned model is used in the third step.

### Embodiment 1:

First step: classify images into the groups of (1) tumors of skin and (2) ones other than the tumors of skin.

Second step: determine the kind of skin tumor in the (1) tumors of skin.

Third step: re-determine the kind of specific skin tumor depending on the result of the second step.

### Embodiment 2:

First step A: exclude images other than skin images.

First step B: classify the images remaining in the first step A into the groups of (1) tumors of skin (solitary skin disease), and (2) ones other than the tumors of skin (including inflammatory skin disease, and normal skin).

Second step: determine the kind of skin tumor in the group of (1) tumors of skin.

Third step: re-determine the kind of specific skin tumor depending on the result of the second step.

### Embodiment 3:

First step A: classify images into the groups of (1) skin diseases (including skin tumor and inflammatory skin disease) and (2) ones other than the skin diseases.

First step B: classify the (1) skin diseases into the groups of (3) tumors of skin and (4) inflammatory diseases.

Second step: determine the kind of skin tumor in the (3) tumors of skin.

Third step: re-determine the kind of specific skin tumor depending on the result of the second step.

### Embodiment 4:

First step: classify images into the groups of (1) skin tumors and (2) ones other than the skin tumors.

Second step A: classify the (1) skin tumors into the groups of (3) melanocytic tumors and (4) amelanotic tumors.

Second step: determine the kind of skin tumor in each of the groups of (3) melanocytic tumors and (4) amelanotic tumors.

Third step: re-determine the kind of specific skin tumor depending on the result of the second step.

### Embodiment 5:

First step: classify images into the groups of (1) skin tumors and (2) ones other than the skin tumors.

Second step: determine the kind of skin tumor in the skin tumors.

Third step (any of the following steps)

Third step 1: in a case of having been determined to be actinic keratosis in the above second step, re-input the image into a determination machine for four kinds of actinic keratosis, actinic keratosis with cutaneous horn, Bowen's disease, and amelanotic seborrheic keratosis, and determine which one of the actinic keratosis, the actinic keratosis with cutaneous horn, the Bowen's disease, and the amelanotic seborrheic keratosis the image is.

Third step 2: in a case of having been determined to be basal cell carcinoma in the above second step, re-input the image into a determination machine for two kinds of basal cell carcinoma and amelanotic basal cell carcinoma, and determine whether the image is the basal cell carcinoma or the amelanotic basal cell carcinoma.

Third step 3: in a case of having been determined to be amelanotic basal cell carcinoma in the above second step, re-input the image into a determination machine for four kinds of amelanotic basal cell carcinoma, actinic keratosis, squamous cell carcinoma, and amelanotic seborrheic keratosis, and determine which one of the amelanotic basal cell carcinoma, the actinic keratosis, the squamous cell carcinoma, and the amelanotic seborrheic keratosis the image is.

Third step 4: in a case of having been determined to be Bowen's disease in the above second step, re-input the image into a determination machine for three kinds of Bowen's disease, actinic keratosis, and amelanotic spitz nevus, and determine which one of the Bowen's disease, the actinic keratosis, and the amelanotic spitz nevus the image is.

Third step 5: in a case of having been determined to be amelanotic poroma in the above second step, re-input the image into a determination machine for three kinds of amelanotic poroma, malignant melanoma, and amelanotic nevocellular nevus, and determine which one of the amelanotic poroma, the malignant melanoma, and the amelanotic nevocellular nevus the image is.

Third step 6: in a case of having been determined to be amelanotic poroma in the above second step, re-input the image into a determination machine for three kinds of amelanotic poroma, malignant melanoma, and basal cell carcinoma, and determine which one of the amelanotic poroma, the malignant melanoma, and the basal cell carcinoma the image is.

Third step 7: in a case of having been determined to be squamous cell carcinoma in the above second step, re-input the image into a determination machine for five kinds of squamous cell carcinoma, actinic keratosis with cutaneous horn, amelanotic basal cell carcinoma, Bowen's disease, and amelanotic malignant melanoma, and determine which one of the squamous cell carcinoma, the actinic keratosis with cutaneous horn, the amelanotic basal cell carcinoma, the Bowen's disease, and the amelanotic malignant melanoma the image is.

Third step 8: in a case of having been determined to be any one of basal cell carcinoma, malignant melanoma, seborrheic keratosis, and nevocellular nevus in the above second step, re-input the image into a determination machine for four kinds of basal cell carcinoma, malignant melanoma, seborrheic keratosis, and nevocellular nevus, and determine which one of the basal cell carcinoma, the malignant melanoma, the seborrheic keratosis, and the nevocellular nevus the image is.

Third step 9: in a case of having been determined to be either seborrheic keratosis or nevocellular nevus in the above second step, re-input the image into a determination machine for four kinds of basal cell carcinoma, malignant melanoma, seborrheic keratosis, and nevocellular nevus, and determine which one of the basal cell carcinoma, the malignant melanoma, the seborrheic keratosis, and the nevocellular nevus the image is.

Third step 10: in a case of having been determined to be either malignant melanoma or nevocellular nevus in the above second step, re-input the image into a determination machine for two kinds of malignant melanoma, and nevocellular nevus, and determine whether the image is the malignant melanoma or the nevocellular nevus.

Third step 11: in a case of having been determined to be nevocellular nevus in the above second step, re-input the image into a determination machine for two kinds of malignant melanoma, and nevocellular nevus, and determine whether the image is the malignant melanoma or the nevocellular nevus.

Third step 12: in a case of having been determined to be amelanotic nevocellular nevus in the above second step, re-input the image into a determination machine for three kinds of amelanotic basal cell carcinoma, amelanotic seborrheic keratosis, and amelanotic nevocellular nevus, and determine which one of the amelanotic basal cell carcinoma, the amelanotic seborrheic keratosis, and the amelanotic nevocellular nevus the image is.

Third step 13: in a case of having been determined to be amelanotic poroma in the above second step, re-input the image into a determination machine for four kinds of basal cell carcinoma, malignant melanoma, seborrheic keratosis, and nevocellular nevus, and determine which one of the basal cell carcinoma, the malignant melanoma, the seborrheic keratosis, and the nevocellular nevus the image is.

In this regard, after the determination of the second step, any one of the third steps 1 to 13 can be appropriately selected and executed. Further, in a case of performing the second step and the third step, the first step may be omitted.

In a preferred embodiment of the present invention, in a case where a tumor class determination step of predicting the kind of skin tumor has been performed, and in a case where the analysis result of the tumor class determination step has been one kind of skin tumors that are easily mistaken for each other, a specific tumor class re-determination step of making a re-determination is executed by a second learned model that has machine learned from images of affected parts of specific skin diseases including the skin tumors that are easily mistaken for each other, and the disease classes described in the above third steps 1 to 12 are examples of a combination of disease classes that are easily mistaken for each other.

In addition, in the above third step 13, amelanotic poroma determined in the second step has not been a candidate for the diseases in the third step, but since the learned model may not have acquired sufficient learning of amelanotic poroma due to the variety of the morphology and the rarity of the cases, the step is an example that can be conveniently used for the purpose of avoiding determining the basal cell carcinoma and malignant melanoma, which are malignant tumors that are erroneously determined to be amelanotic poroma, to be benign (false negative) as much as possible.

### Example 1

In the present Example, as the classifier, two models were selected from known image classification models carrying a lot of weight learned by ImageNet, and the models were each allowed to learn by using 7532 images in total of teacher images (images of skin tumors of the above 24-class) (an example of a first-learned model). The breakdown of the images is as follows: as malignant tumors, 278 images of actinic keratosis (AK), 95 images of actinic keratosis with cutaneous horn (AKhorn), 419 images of Bowen's disease (Bowen), 1166 images of squamous cell carcinoma (SCC), 1100 images of basal cell carcinoma (BCC), 284 images of amelanotic basal cell carcinoma (BCCamela), 335 images of extramammary Paget's disease (EMPD), 625 images of malignant melanoma (MM), 74 images of amelanotic malignant melanoma (MMamela), and 81 images of angiosarcoma (AS); and as benign tumors, 103 images of poroma (Poroma), 87 images of amelanotic poroma (Poromamela), 96 images of sebaceous nevus (SebaceousN), 520 images of seborrheic keratosis (SK), 66 images of amelanotic seborrheic keratosis (SKamela), 119 images of blue nevus (BlueN), 238 images of congenital melanocytic nevus (CongenitalN), 1165 images of nevocellular nevus (NCN), 133 images of amelanotic nevocellular nevus (NCNamela), 204 images of spitz nevus (Spitz), 36 images of amelanotic spitz nevus (Spitzamela), 42 images of lentigo (Lentigo), 161 images of nevus spilus (Spilus), and 105 images of pyogenic granuloma (PG). All of these images were diagnosed by a specialist of skin tumor, and photographed after the confirmation of the diseases.

By dividing the image into 10 parts and using 9/10 parts for teacher (learning) images and a 1/10 part for a test image, the performance of a learning machine was evaluated with the use of a cross-validation method.

According to the present Example, the percentage of overall correct answers was 68% in Model 1, and was 69% in Model 2. In Model 2, the determination results were as follows: 80 images were correctly determined to be squamous cell carcinoma, 4 images were determined to be actinic keratosis. 4 images were determined to be Bowen's disease, 5 images were determined to be basal cell carcinoma, 7 images were determined to be amelanotic basal cell carcinoma, 2 images were determined to be extramammary Paget's disease, 1 image was determined to be malignant melanoma, 1 image was determined to be seborrheic keratosis, 4 images were determined to be amelanotic nevocellular nevus, and 1 image was determined to be pyogenic granuloma, among 110 images of squamous cell carcinoma, by the classifier. That is, the proportion (sensitivity: Recall) of correctly determining the images of squamous cell carcinoma to be squamous cell carcinoma was 73%.

In addition, among 109 images of affected parts, which had been determined to be squamous cell carcinoma by the classifier, 80 images were images of the actual diseases determined to be squamous cell carcinoma, and actually, 1 image was determined to be actinic keratosis, 4 images were determined to be actinic keratosis with cutaneous horn, 4 images were determined to be Bowen's disease, 5 images were determined to be basal cell carcinoma, 5 images were determined to be amelanotic basal cell carcinoma, 1 image was determined to be malignant melanoma, 1 image was determined to be amelanotic malignant melanoma, 4 images were determined to be poroma, 3 images were determined to be amelanotic poroma, and 1 image was determined to be seborrheic keratosis. That is, among the images determined to be squamous cell carcinoma, the images of the actual diseases determined to be squamous cell carcinoma (positive predictive value: Precision) was 73%.

### (TOP3 of percentage of correct answers of 24-class classification)

In the 24-class disease determination, the classifier determined the highly probable top 3 kinds of skin tumors. At this time, the determination results (percentage of correct answers) when the case where there had been a correct answer in any of the three kinds determined by the classifier was defined to have a TOP3-correct answer of 24-class classification were 91% in Model 1 and 89% in Model 2.

### (TOP3 of percentage of correct answers of 2-class classification)

In the 24-class disease determination, the classifier determined the highly probable top 3 kinds of skin tumors. At this time, in a case where the actual tumor was malignant tumor and there was a malignant tumor in any of the three kinds determined by the classifier, the case was taken to have a correct answer for malignant determination. Further, in a case where the actual tumor had been benign tumor and all of the three kinds determined by the classifier had been benign tumors, the case was taken to have a correct answer for benign determination. The determination results (percentage of correct answers) when the case where this was defined to have a TOP3-correct answer of 2-class classification were 99% in Model 1 and 98% in Model 2.

### (Sensitivity of 2-class classification)

In the 24-class disease determination, the classifier determined the most highly probable kind of skin tumor. When the skin tumor that had been determined to be most highly probable by the classifier was determined to be benign or malignant, the sensitivity (Recall) of the determination results was 94% for malignant and 79% for benign in Model 1, and was 94% for malignant and 83% for benign in Model 2.

### Example 2

As the classifier, two models were selected from known image classification models carrying a lot of weight learned by ImageNet, and the models were each allowed to learn by using 7011 images in total of teacher images (images of skin tumors of 21-class excluding 3 classes of extramammary Paget's disease, angiosarcoma, and pyogenic granuloma from the 24-class).

By dividing the image into 10 parts and using 9/10 parts for teacher images and a 1/10 part for a test image, the performance of a learning machine was evaluated with the use of a cross-validation method.

According to the present Example, the percentage of overall correct answers was 70% in Model 1 and was 74% in Model 2. In Model 2, the determination results were as follows: 64 images were correctly determined to be malignant melanoma, 1 image was determined to be squamous cell carcinoma, 4 images were determined to be basal cell carcinoma, 2 images were determined to be amelanotic malignant melanoma, and 2 images were determined to be seborrheic keratosis, among 72 images of malignant melanoma, by the classifier. That is, the proportion (sensitivity: Recall) of correctly determining the images of malignant melanoma to be malignant melanoma was 83%.

In addition, among 86 images of affected parts, which had been determined to be malignant melanoma by the classifier, 64 images were images of the actual diseases determined to be malignant melanoma, 1 image was an image of the actual diseases determined to be actinic keratosis, 2 images were determined to be squamous cell carcinoma, 3 images were determined to be basal cell carcinoma, 1 image was determined to be amelanotic malignant melanoma, 3 images were determined to be amelanotic poroma, 3 images were determined to be seborrheic keratosis, 4 images were determined to be blue nevus, 2 images were determined to be congenital melanocytic nevus, 2 images were determined to be nevocellular nevus, and 2 images were determined to be spitz nevus. That is, among the images determined to be malignant melanoma, the images of the actual diseases determined to be malignant melanoma (positive predictive value: Precision) was 74%.

### (TOP3 of percentage of correct answers of 21-class classification)

In the 21-class disease determination, the classifier determined the highly probable top 3 kinds of skin tumors. At this time, the determination results (percentage of correct answers) when the case where there had been a correct answer in any of the three kinds determined by the classifier was defined to have a TOP3-correct answer of 21-class classification were 93% in Model 1 and 93% in Model 2.

### (TOP3 of percentage of correct answers of 2-class classification)

In the 21-class disease determination, the classifier determined the highly probable top 3 kinds of skin tumors. At this time, in a case where the actual tumor was malignant tumor and there was a malignant tumor in any of the three kinds determined by the classifier, the case was taken to have a correct answer for malignant determination. Further, in a case where the actual tumor had been benign tumor and all of the three kinds determined by the classifier had been benign tumors, the case was taken to have a correct answer for benign determination. The determination results (percentage of correct answers) when the case where this was defined to have a TOP3-correct answer of 2-class classification were 99% in Model 1 and 98% in Model 2.

### (Sensitivity of 2-class classification)

In the 21-class disease determination, the classifier determined the most highly probable kind of skin tumor. When the skin tumor that had been determined to be most highly probable by the classifier was determined to be benign or malignant, the sensitivity (Recall) of the determination results was 98% for malignant and 84% for benign in Model 1, and was 97% for malignant and 88% for benign in Model 2.

### Example 3

Model experiment 1 of elimination determination device (first step):
For example, an elimination determination device (model for exclusion determination) was created by utilizing a deep neural network as a feature extractor, with a procedure of, for example, making an exclusion determination by using the features.

Six images that were not images of skin tumors were input to the elimination determination device.

As a result, all of the 6 images (car, cat, globe, arm, dog, and boat) were excluded as not being skin tumor.

At this time, around 5% of the images of skin tumors were excluded as not being skin tumor.

Further, when six images that had been excluded as not being skin tumor were input into a classifier of Model 1, the disease that was determined to be most probable was malignant melanoma in all of the 6 images. This indicates that the classifier determines an image to be any one of classes of skin tumor, even if the image has nothing to do with skin tumor.

### Example 4

Model experiment 2 of elimination determination device (first step):
As the classifier, one model was selected from known image classification models carrying a lot of weight learned by ImageNet, and the models were each allowed to learn by using 7011 images in total of teacher images (images of skin tumors of the same 21-class as in the above Example 2), and 1044 images in total of atopic dermatitis and psoriasis, which are inflammatory skin diseases, and mycosis fungoides of 3-class. In the learning, by dividing the image into 10 parts and using 9/10 parts for teacher images and a 1/10 part for a test image, the generalization accuracy was calculated with test data.

As the test images, 691 images of skin tumors, and 74 images of inflammatory skin diseases were used. As a result, 689 images of skin tumors were determined to be skin tumor by the classifier, and 2 images of skin tumors were determined to be inflammatory skin disease by the classifier. Further, 66 images of inflammatory skin diseases were determined to be inflammatory skin disease by the classifier, and 8 images of inflammatory skin diseases were determined to be skin tumor by the classifier. That is, from the viewpoint of skin tumor, the sensitivity (Recall) was 99.7%, and the positive predictive value (precision) was 98.9%.

Accordingly, in a case where an image of skin disease was input, the image can be determined whether it indicates skin tumor, or inflammatory skin disease. Therefore, according to this elimination determination device, by inputting an image that is certain to be some kind of skin disease, it becomes possible to exclude the inflammatory skin disease, determine only the skin tumor, and use the image in the next second step.

### Example 5

Model experiment 3 of elimination determination device (first step):
As the classifier, one model was selected from known image classification models carrying a lot of weight learned by ImageNet. Further, 7011 images in total of skin tumors of the same 21-class as in the above Example 2 were used as the images of skin tumors. In addition, 7000 images randomly selected from confirmation data of Google Open Image V4 were used as images other than the images of skin tumors. By using 3476 images of skin tumors and 3500 images other than the images of skin tumors as the teacher data among the above images, the learning was performed by deep learning.

In addition, for remaining 3575 images of skin tumors and 3500 images other than the images of skin tumors, it was determined whether or not each of the images was a skin tumor by the learned model.

As a result, among the 3575 images of skin tumors, 3567 images were determined to be skin tumor, and 8 images were determined not to be skin tumor. Further, among the 3500 images of not being skin tumor, 19 images were determined to be skin tumor, and 3481 images were determined not to be skin tumor.

That is, from the viewpoint of skin tumor, the sensitivity was 99.8%, the specificity was 99.5%, the positive predictive value was 99.8%, the negative predictive value was 99.5%, and the percentage of correct answers was 99.6%.

Even in a case where an image other than the images of skin tumors has been transmitted to a classifier due to a mistake in transmission or the like, the model of this elimination determination device can detect that the image is not a skin tumor, notify the user to that effect, and avoid the erroneous determination.

### Example 6

In the present Example, as the classifier, one model was selected from known image classification models carrying a lot of weight learned by ImageNet. As the teacher image and the test image, 7532 images in total of skin tumors of the above 24-class were used in a similar manner as in Example 1.

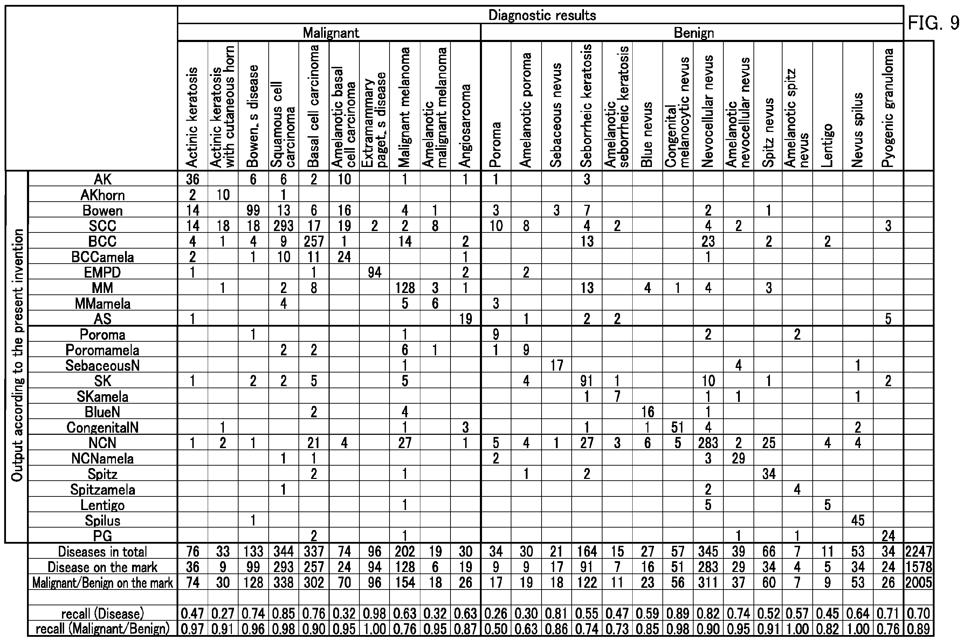

By dividing the image into 10 parts, allowing a learning machine to learn by using 9/10 parts for teacher images, and using a 1/10 part for a test image, the performance of the learning machine was evaluated with the use of a cross-validation method. As a result of performing this cross-validation three times, the number of images determined as the test images was 2247 in total three times. The determination results are shown in Fig. 9.

According to the results, among the 2247 images, the predictive value of the images correctly determined as the disease for each disease was 70%. Further, the predictive value of the images correctly determined as to whether the disease is malignant or benign was 89%.

Clinically, it is required not to mistake a malignant tumor for a benign tumor, and there is a characteristic tendency even in a case where a malignant tumor has been erroneously determined to be a benign tumor. From the determination results shown in Figs. 9 and 29, it can be understood that there are many cases in which the tumors are mistaken for each other in 4-class of basal cell carcinoma (BCC), malignant melanoma (MM), seborrheic keratosis (SK), and nevocellular nevus (NCN). Further, it can be understood that malignant melanoma (MM) is often erroneously determined to be benign amelanotic poroma (Poromamela) in addition to the above 3-class. As described above, in a case where an image has been determined to be a tumor that is easily erroneously determined in 24-class determination, it is preferable to re-determine the image by using a classifier created by narrowing the images to be input down to images of the tumor class in which the tumors are easily mistaken for each other.

### Example 7

Next, in view of this, as shown in Fig. 9 and Fig. 29 to be described later, a classifier was created by allowing a model to learn by using only the images indicating 4 tumors of basal cell carcinoma, malignant melanoma, seborrheic keratosis, and nevocellular nevus that are easily mistaken for each other (an example of a second-learned model). As the classifier used here, one model was selected from known image classification models carrying a lot of weight learned by ImageNet. Further, the images used were 1100 images of basal cell carcinoma, 625 images of malignant melanoma, 520 images of seborrheic keratosis, and 1165 images of nevocellular nevus.

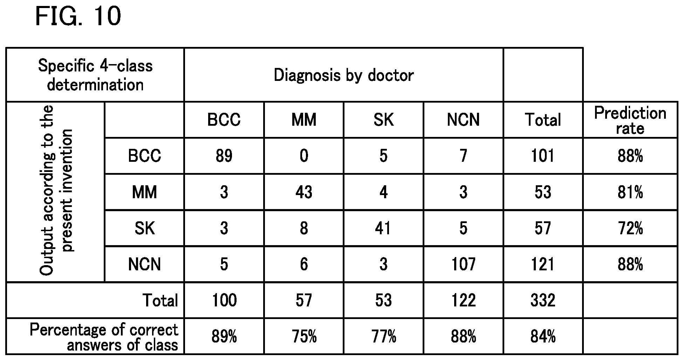

By dividing each of the above images of 4-tumor class (1100 images of basal cell carcinoma, 625 images of malignant melanoma, 520 images of seborrheic keratosis, and 1165 images of nevocellular nevus) into 10 parts, and using 9/10 parts for teacher images and a 1/10 part for a test image, the evaluation was performed with the use of a cross-validation method. This evaluation was performed 10 times, and the results that were obtained by making the most average determination among the evaluations are shown in Fig. 10.

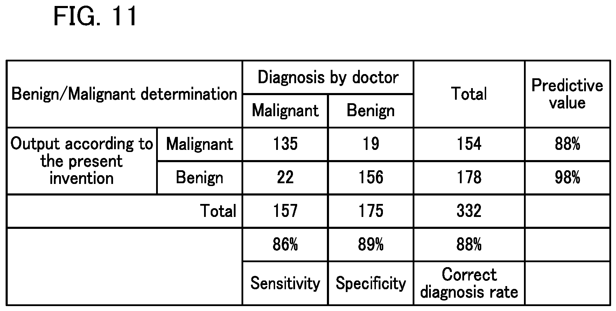

In addition, the determination results as the malignant (basal cell carcinoma and malignant melanoma) and the benign (seborrheic keratosis and nevocellular nevus) are shown in Fig. 11.

### Example 8

By dividing each of the above images of 4-tumor class into 10 parts and using 9/10 parts for teacher images and a 1/10 part for a test image, the evaluation was performed with the use of a cross-validation method. In this regard, in the present Example, from the images indicating basal cell carcinoma and malignant melanoma, images indicating diseases similar to other diseases were extracted (for example, images determined to be other disease classes in 24-class determination and images in the same cases as those of the above images determined to be other disease classes), and 292 images of basal cell carcinoma and 149 images of malignant melanoma were selected.

The number of these images were increased 10-fold by performing processing such as changing the contrast, or adding noise. As a result, in the images of basal cell carcinoma used in the present Example, there were 2910 images of the 10-fold increase, and 808 images that had not been subjected to the above processing, and thus the total number of the images was 3718. Further, in the images of malignant melanoma used in the present Example, there were 1490 images of the 10-fold increase, and 456 images that had not been subjected to the above processing, and thus the total number of the images was 1946. In this regard, for the images of affected parts of malignant melanoma (melanoma), which were similar in shape to the images of nevocellular nevus, it is preferable to perform the processing so as to increase the number of the images at least 2 or more-fold, and it is more preferable to perform the processing so as to increase the number of the images 5 or more-fold. In addition, in malignant melanoma and/or basal cell carcinoma, for the images of affected parts, which were similar in shape to the images of other diseases, it is preferable to perform the processing so as to increase the number of the images at least 2 or more-fold, and it is more preferable to perform the processing so as to increase the number of the images 5 or more-fold.

Further, the number of the images of seborrheic keratosis used in the present Example was 520, and the number of the images of nevocellular nevus used in the present Example was 1165.

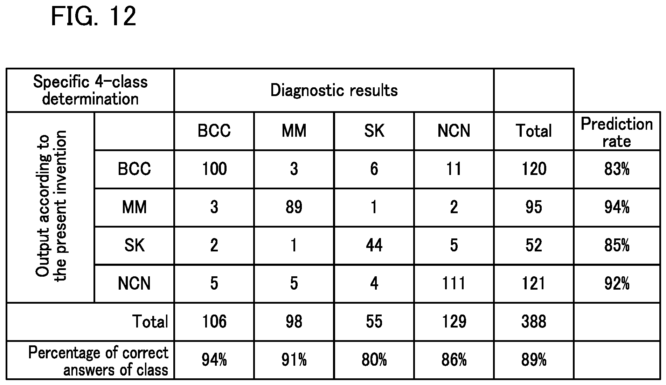

This evaluation was performed 10 times with the use of a 10-fold cross-validation method, and the evaluation results that were obtained by making the most average determination among the evaluations are shown in Fig. 12.

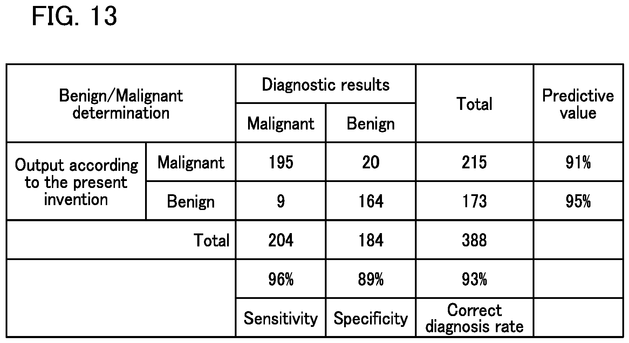

In addition, the determination results as the malignant (basal cell carcinoma and malignant melanoma) and the benign (seborrheic keratosis and nevocellular nevus) are shown in Fig. 13.

As a result, when comparing Example 7 (Figs. 10 and 11) with Example 8 (Figs. 12 and 13), the higher accuracy was obtained in Example 8. In Example 8, it is considered that by increasing the number of the images that are easily mistaken for images of other disease classes, the images that are easily mistaken for images of other disease classes are abundantly learned, and as a result, the diseases that are similar to each other in the image can be more accurately determined.

### Example 9

In the present Example, the images of which the determination results by the 24-class classifier used in Example 6 had been each basal cell carcinoma, malignant melanoma, seborrheic keratosis, or nevocellular nevus were re-determined by the 4-class classifier used in Example 8, and the results were simulated.

The procedure is as follows.

Among the images used in Example 6, images of which the output results by the 24-class classifier in Example 6 had each indicated basal cell carcinoma, malignant melanoma, seborrheic keratosis, or nevocellular nevus (all of correct answers and incorrect answers) were extracted from the images that had each indicated basal cell carcinoma, malignant melanoma, seborrheic keratosis, or nevocellular nevus in the actual diagnostic results.

Among the extracted images, images used in Example 8 were extracted. That is, the images used in both Examples were extracted.

Further, for each of the extracted images, first, the output result was determined by the 24-class classifier to be basal cell carcinoma, malignant melanoma, seborrheic keratosis, or nevocellular nevus, and then re-determined by the 4-class classifier (used in Example 8), and the results were obtained.

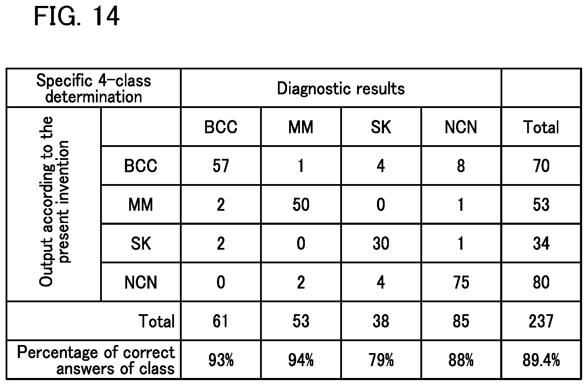

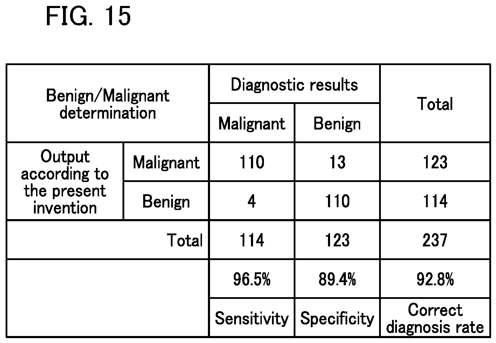

The results are shown in Fig. 14. In addition, the determination results as the malignant (basal cell carcinoma and malignant melanoma) and the benign (seborrheic keratosis and nevocellular nevus) are shown in Fig. 15.

### Example 10

In the present Example, the images of which the determination results by the 24-class classifier used in Example 6 had been each seborrheic keratosis, or nevocellular nevus were re-determined by the 4-class classifier used in Example 8, and the results were simulated.

The procedure is as follows.

Among the images used in Example 6, images of which the output results by the 24-class classifier in Example 6 had each indicated basal cell carcinoma, malignant melanoma, seborrheic keratosis, or nevocellular nevus (all of correct answers and incorrect answers) were extracted from the images that had each indicated basal cell carcinoma, malignant melanoma, seborrheic keratosis, or nevocellular nevus in the actual diagnostic results.

Among the extracted images, images used in Example 8 were extracted. That is, the images used in both Examples were extracted.

Further, for each of the extracted images, first, the output result was determined by the 24-class classifier to be basal cell carcinoma, malignant melanoma, seborrheic keratosis, or nevocellular nevus, and then the images of which the output results had been seborrheic keratosis or nevocellular nevus were re-determined by the 4-class classifier (used in Example 8), and the results were obtained. In this regard, for the image determined to be basal cell carcinoma or malignant melanoma by the 24-class classifier at this time, the result was maintained and the re-determination by the 4-class classifier was not performed.

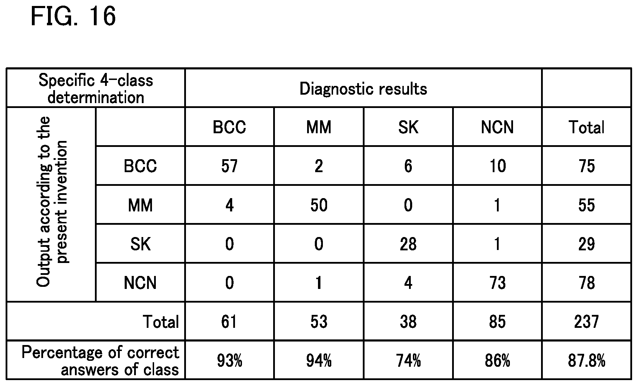

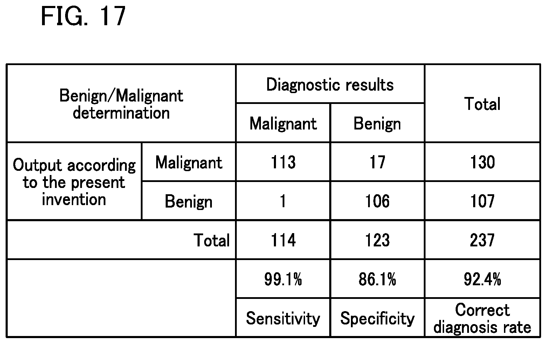

The results are shown in Fig. 16. In addition, the determination results as the malignant (basal cell carcinoma and malignant melanoma) and the benign (seborrheic keratosis and nevocellular nevus) are shown in Fig. 17.

As shown in Fig. 17, by making a re-determination of benign tumors that are easily erroneously determined to be specific malignant tumors, the number of cases where a benign tumor is erroneously determined to be a malignant tumor increases slightly, but the number of cases where a malignant tumor is erroneously determined to be a benign tumor decrease, and thus such a re-determination is useful from the viewpoint of not overlooking any malignant tumor. Reference Example 1

As a result of the determination of images of various kinds of tumor classes by the 24-class classifier, the images of which the outputs each indicated basal cell carcinoma, malignant melanoma, seborrheic keratosis, or nevocellular nevus also include an image of which the actual diagnostic result was not the basal cell carcinoma, malignant melanoma, seborrheic keratosis, or nevocellular nevus (see Figs. 9 and 29).

In view of this, in the present Reference Example, among the images of which the outputs had been each basal cell carcinoma, malignant melanoma, seborrheic keratosis, or nevocellular nevus by the 24-class classifier, the images that had not indicated any of these tumors of the 4-class in the actual diagnostic results were each re-determined by the 4-class determination device, and the results were simulated.

Among the images used in Example 6, images of which the output results by the 24-class classifier in Example 6 had each indicated basal cell carcinoma, malignant melanoma, seborrheic keratosis, or nevocellular nevus (in this case, all of which are incorrect answers) were extracted from the images that had each not indicated basal cell carcinoma, malignant melanoma, seborrheic keratosis, or nevocellular nevus in the actual diagnostic results.

Among the extracted images, images used in Example 8 were extracted. That is, the images used in both Examples were extracted.

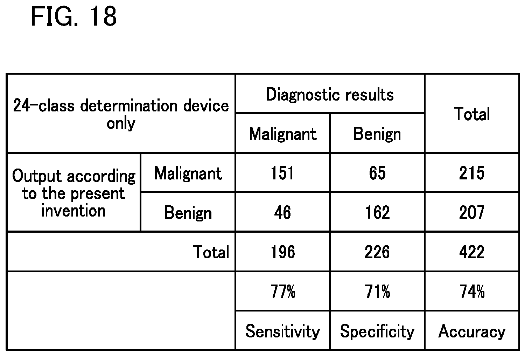

Further, for each of the extracted images, first, the output result was determined by the 24-class classifier to be basal cell carcinoma or malignant melanoma (malignant tumor), or seborrheic keratosis or nevocellular nevus (benign tumor). The determination results of malignant or benign are shown in Fig. 18.

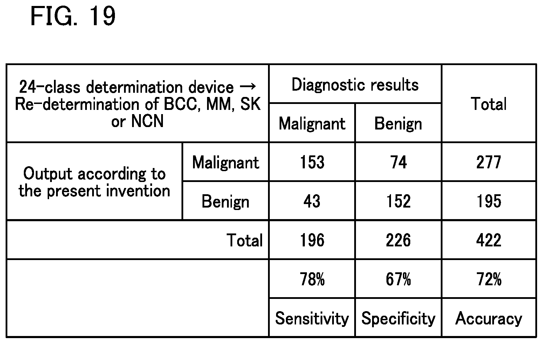

Next, the extracted images were each re-determined by the 4-class classifier (used in Example 8), and the results were obtained. The determination results of malignant or benign are shown in Fig. 19.

### Reference Example 2

In the present Reference Example, among the images of which the outputs had been each seborrheic keratosis or nevocellular nevus by the 24-class classifier, the images that had not indicated any of these tumors of 4-class of basal cell carcinoma, malignant melanoma, seborrheic keratosis, and nevocellular nevus in the actual diagnostic results were re-determined by the 4-class determination device, and the results were simulated.

Among the images used in Example 6, images of which the output results by the 24-class classifier in Example 6 had each indicated seborrheic keratosis or nevocellular nevus (in this case, all are incorrect answers) were extracted from the images that had each not indicated basal cell carcinoma, malignant melanoma, seborrheic keratosis, or nevocellular nevus in the actual diagnostic results.

Among the extracted images, images used in Example 8 were extracted. That is, the images used in both Examples were extracted.

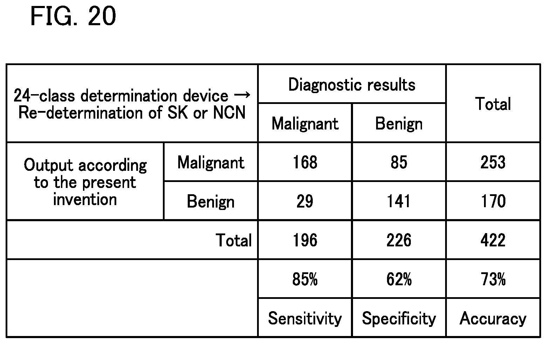

Further, for each of the extracted images, first, the output result was determined by the 24-class classifier to be basal cell carcinoma or malignant melanoma (malignant tumor), or seborrheic keratosis or nevocellular nevus (benign tumor). Next, the images of which the determination results had been each seborrheic keratosis or nevocellular nevus were each re-determined by the 4-class classifier (used in Example 8), and the results were obtained. The results of re-determination of malignant or benign are shown in Fig. 20.

As a result of Reference Examples 1 and 2, the accuracy of tumors of the kinds not belonging to any of the specific 4-class almost remained unchanged even in a case where the 4-class classifier was used, and thus, it is considered that even if the 4-class classifier is used for tumors other than those of 4-class, there is no adverse effect caused by this.

### Example 11

In the present Example, multiple photographs are taken for one case, and the effect of the determination of the multiple photographs is confirmed in a case where images of the photographs are used in the 24-class classifier.

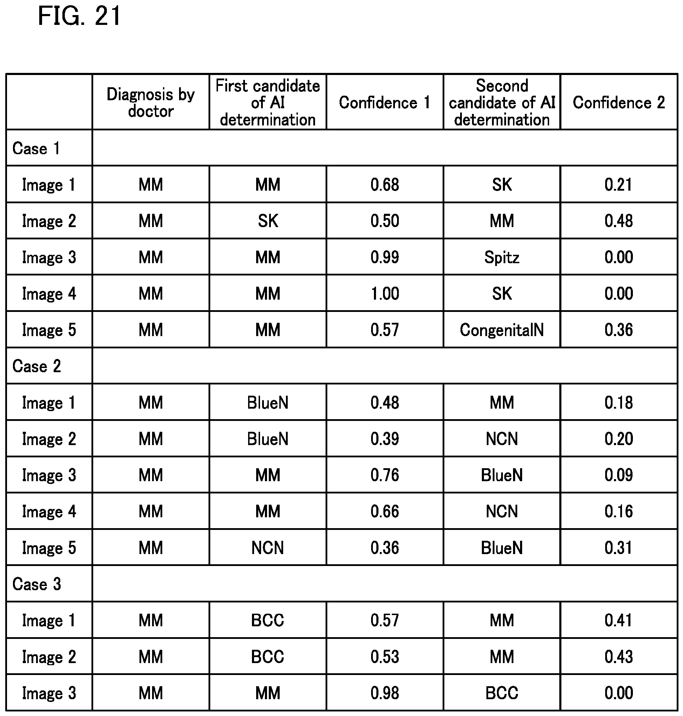

Among the images used in Example 6, some images are multiple images for one case. All of the multiple images for the same case may indicate the same determination results, but the determination results may be different from each other in some cases. Fig. 21 shows three cases that have multiple image data for the case diagnosed with malignant melanoma and have different determination results (as the first candidate of AI determination).

When the 24-class classifier used in Example 6 makes a determination, the 24-class classifier outputs a numerical value so that the numerical value becomes 1 by adding the certainty (confidence) determined by the classifier for each of the tumors of 24-class. The top two tumors with high confidence are shown in Fig. 21. Usually, the tumor with the highest confidence is used as the determination result of the classifier.

Referring to Fig. 21, the confidence may vary even in the same case, and further, the tumor with the highest confidence (that is, determination result) may be different in some cases.

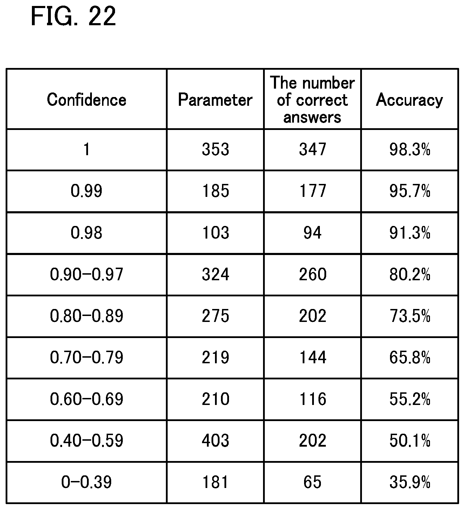

In addition, in Example 6, when referring to the obtained determination results, it was found that the accuracy differs depending on the degree of confidence at that time (corresponding to Confidence 1 in Fig. 21). That is, as shown in Fig. 22, the result that the higher the confidence, the higher the accuracy was obtained. This suggests that the accuracy can be improved by determining multiple images for one case, and adopting the image with the highest confidence after each determination of the multiple images.

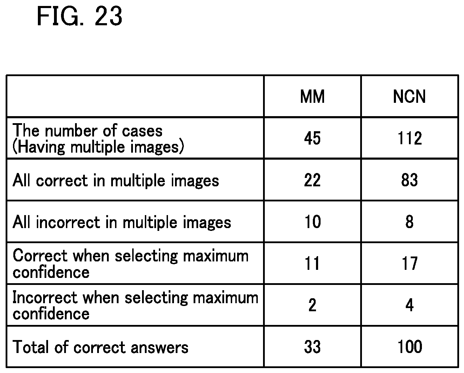

In Fig. 23, for malignant melanoma (MM) and nevocellular nevus (NCN), an example in which multiple images were determined for the same case in Example 6 is shown.

The expression "all correct in multiple images" means that all of the multiple images for the same case indicated correct answers when the images were determined (for example, the determination results by AI were all MM in a case where the diagnostic result was MM).

The expression "all incorrect in multiple images" means that all of the multiple images for the same case indicated incorrect answers when the images were determined.

The expression "correct when selecting maximum confidence" means that when the multiple images for the same case were determined, correct and incorrect answers were mixed, and when an image with the highest confidence was selected from the images, the image was correct.

The expression "incorrect when selecting maximum confidence" means that when the multiple images for the same case were determined, correct and incorrect answers were mixed, and when an image with the highest confidence was selected from the images, the image was incorrect.

In the present Example, as the classifier, one model was selected from known image classification models carrying a lot of weight learned by ImageNet. As the teacher image and the test image, 7532 images in total of skin tumors of the above 24-class were used in a similar manner as in Example 1.

As shown in Fig. 23, in both cases of malignant melanoma and nevocellular nevus, it was indicated that the number of "correct when selecting maximum confidence" is considerably larger than that of "incorrect when selecting maximum confidence", that is, the accuracy can be expected to improve by photographing multiple images.

### Example 12

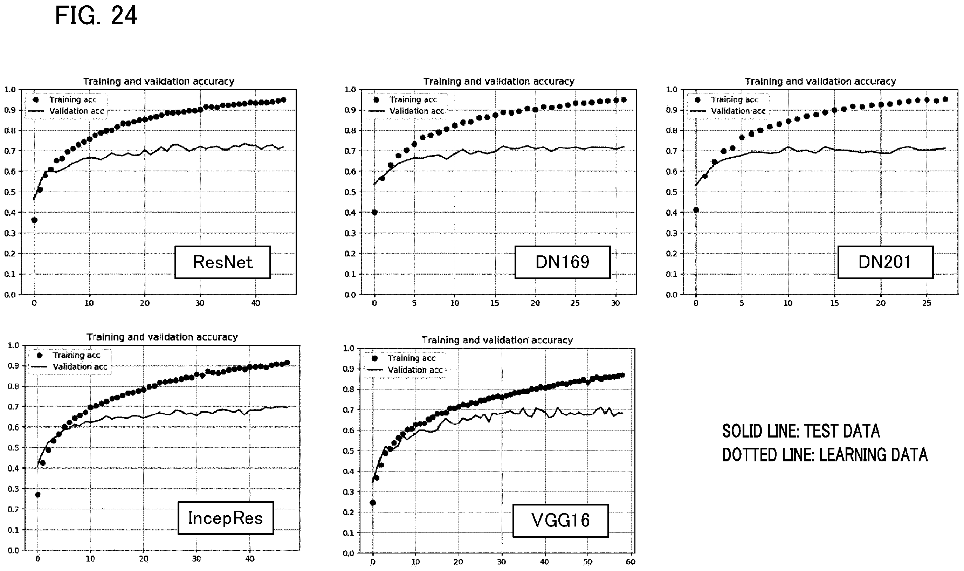

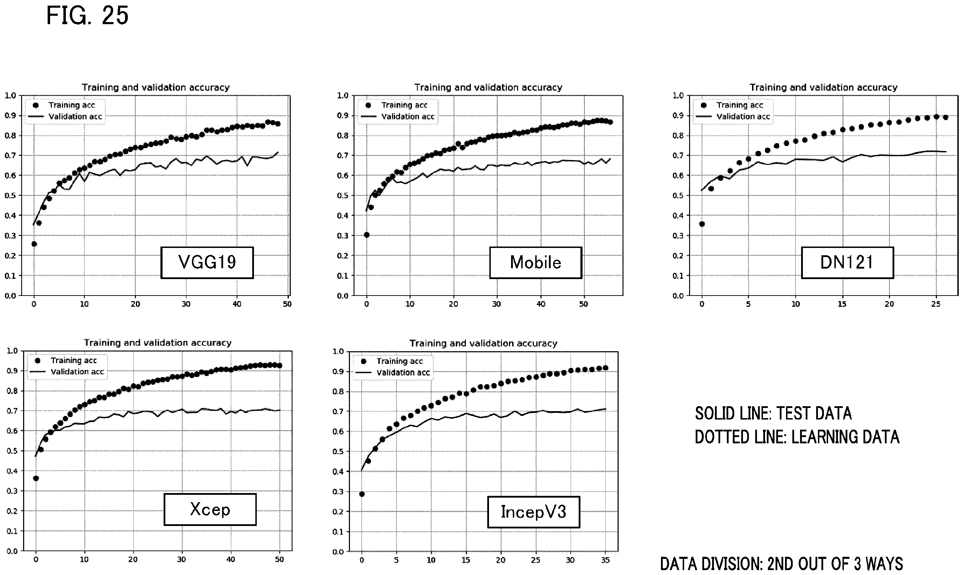

In the present Example, as the classifier, 10 models of known image classification models carrying a lot of weight learned by ImageNet (ResNet50, DenseNet169, DenseNet201, InceptionResNetV2, VGG16, VGG19, MobileNet, DenseNet121, Xeption, and InceptionV3), were each allowed to learn and tested by using teacher images (7532 images in total of skin tumors in the above 24-class). By using around 90% of images as learning data, and around 10% of images as test data, determination of the learning data and determination of the test data were performed. In the learning of the models, re-learning was performed so that the number of correct answers in the learning data increases, and the re-learning was repeated until the accuracy of the test data did not increase. The learning curves up to the time point when the re-learning was finished are shown in Figs. 24 and 25. In Figs. 24 and 25, the vertical axis represents the accuracy and the horizontal axis represents the number of times of re-learning.

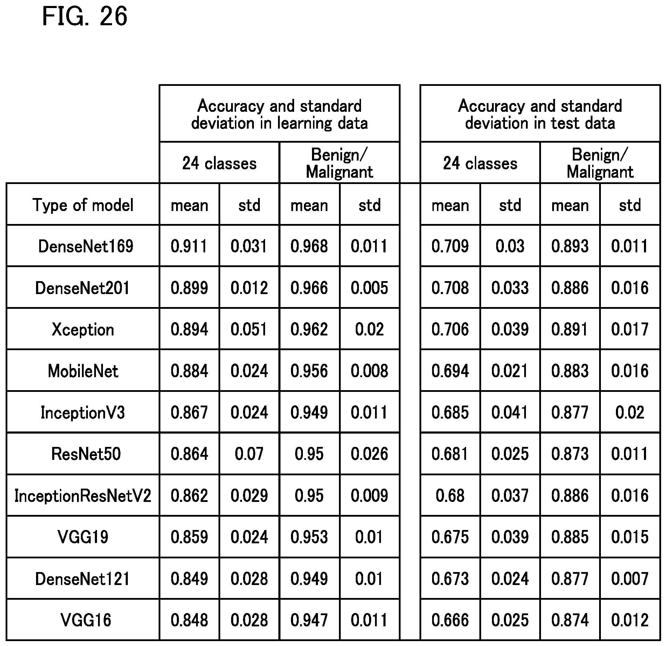

Further, the accuracies for the models with the highest accuracies for the test data at the time point when the re-learning was finished are shown in Fig. 26. In this case, the test was performed three times, and the average and standard deviation of the accuracies are shown for the 24-class determination and the benign/malignant determination. It is understood that a certain favorable accuracy can be obtained in any of the learned models.

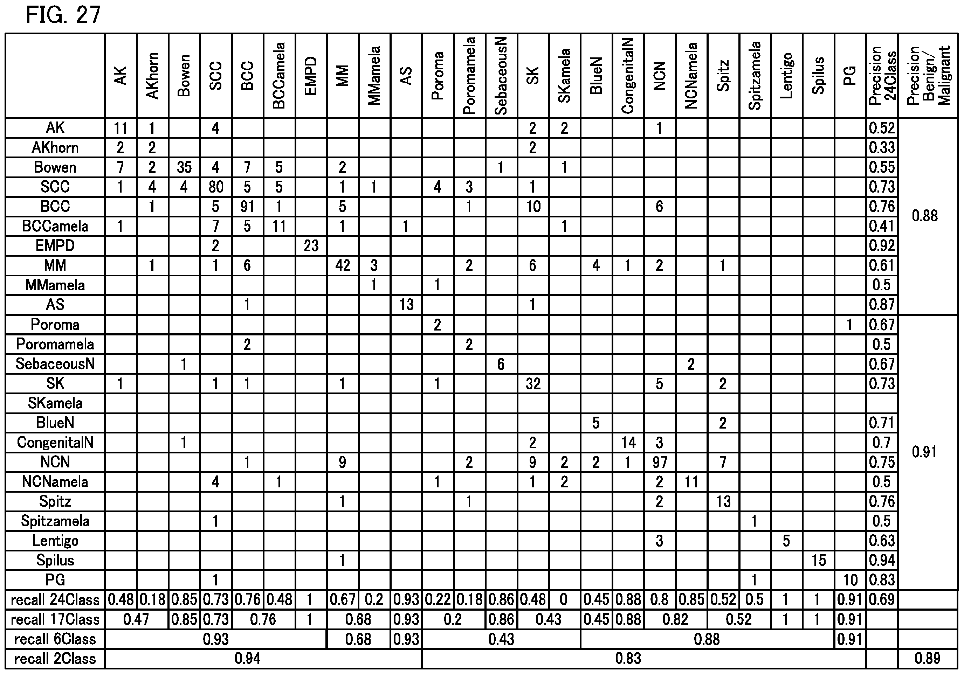

In addition, an example of a confusion matrix of test data in a case where DenseNet201 was used as the model is shown in Fig. 27. In this case, 752 images were used as test data.

From the viewpoint of distinguishing between malignant and benign, the overall accuracy was 89%, the accuracy for malignant tumor was 94%, and the accuracy for benign tumor was 83%.

In Fig. 27, recall 17Class shows the accuracies when the 24-class has been summarized into 17-class. For example, BCC (basal cell carcinoma) and BCCamela (amelanotic basal cell carcinoma) are summarized in the same class. Accordingly, if the diagnostic result was BCC, the estimation result of the system according to the present invention is admitted to be correct when it indicated BCC or BCCamela.

Further, in Fig. 27, recall 6Class shows the results when the 24-class has been summarized into 6-class. For example, AK, AKhorn, bowen, SCC, BCC, BCCamela, and EMPD (these are classified into malignant tumor of epitheliocytes) have been summarized in the same class. Accordingly, if the diagnostic result was AK, the estimation result of the system according to the present invention is admitted to be correct when it indicated AK, AKhorn, bowen, SCC, BCC, BCCamela, or EMPD.

In implementing these models, an optimum model can be appropriately selected in consideration of, for example, the tendency of tumors to be mistaken, the susceptibility to over learning, and what kind of determination result to be output.

### Example 13

In the present Example, as the classifier, one model was selected from known image classification models carrying a lot of weight learned by ImageNet. As the teacher image and the test image, 7532 images in total of skin tumors of the above 24-class were used in a similar manner as in Example 1.

By randomly extracting roughly a 1/10 part of the image each time to use it for a test image, and allowing a learning machine to learn by using roughly 9/10 parts for learning images, the performance of the learning machine was evaluated with the use of a cross-validation method. As a result of performing this cross-validation ten times, the number of images determined as the test images was 7472 in total ten times. The determination results are shown in Fig. 29.

From the viewpoint of predicting malignant or benign, the overall accuracy was 90.0%, the accuracy for malignant tumor was 92.7%, and the accuracy for benign tumor was 85.8%.

### Example 14

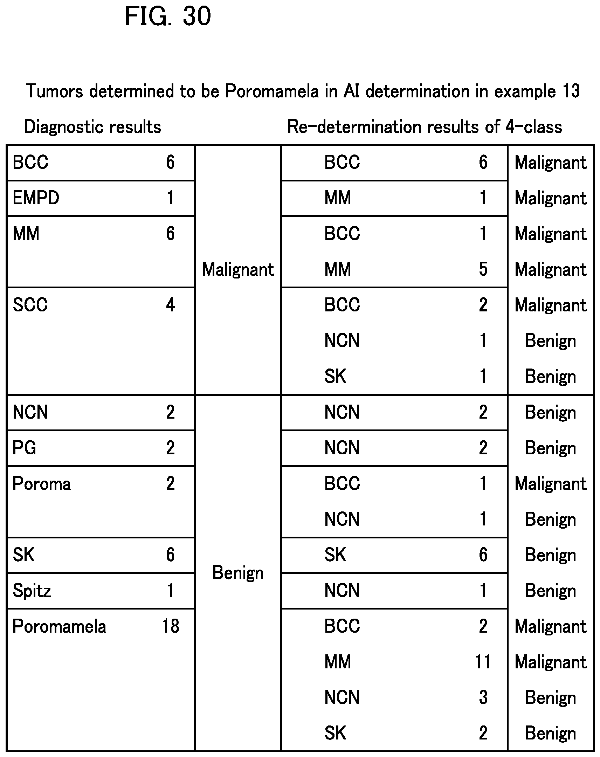

In the 24-class determination of Example 13, 48 images were determined to be poroma (Poromamela). These 48 images were re-determined by the 4-class determination device (that determines which one of BCC, MM, SK, and NCN) used in Example 8. The results are shown in Fig. 30. Among the images determined to be poromamela, 17 images were malignant tumor, and 15 images out of the 17 images were determined to be malignant (correct answer) in the 4-class re-determination. In addition, among 31 images that were determined to be benign, 14 images were determined to be malignant (incorrect answer) in the 4-class re-determination. The overall accuracy in the 4-class re-determination was 67%, and was almost the same as that in the 24-class determination (65%). According to this, from the viewpoint of not overlooking any malignancy, it is considered that the re-determination of specific tumor class is effective.

### Example 15

In the present Example, three of an excluded determination engine (first step), a 24-class determination device (second step), and a 4-class re-determiner (third step) were implemented as the classifier, and the determination was performed by using 4 images at the maximum for one case.

As the user client terminal, iPod (registered trademark) touch MVHW2J/A was used. The client terminal is loaded with an application having functions of performing login authentication of a user for logging in there, photographing 4 images at the maximum by using a camera function of iPod totch accessed through a web browser, providing a guide frame so that a tumor is placed in the central part of a photograph at the time of taking the photograph (cutting out the affected part), and sending the taken photographs to a server. As the browser, Google Chrome was used. For the communication between the client terminal and the server, for example, for sending a photograph to a server, WiFi was used.

Further, as the server, a server with the following specifications was used. CPU: Intel (registered trademark) Core i7-8700K, 3.70 GHz, GPU: GeForce GTX 1080 Ti, and Memory: 32GB.

In the server, first, the application server receives the photographs sent from a client terminal, and in the AI server, each of the photographs is determined by (1) a learned model determining whether or not the photograph indicates a tumor image (exclusion determination), (2) a learned model determining which one of the diseases in 24-disease class, and (3) a learned model determining which one of BCC, MM, SK, and NCN of 4-disease class the disease is. In this regard, at the determination, the determination result is indicated by the name of the disease class and the confidence (the total is 1) as to which disease class the determination result corresponds to. In addition, the encrypted photographs and the determination results are stored in the file server.

The determination results can be referred from the manager client terminal.

When a photograph of an affected part was taken and determined by using the client terminal and the server, the time from the transmission of 4 photographs to the completion of determination was approximately 60 to 120 seconds.

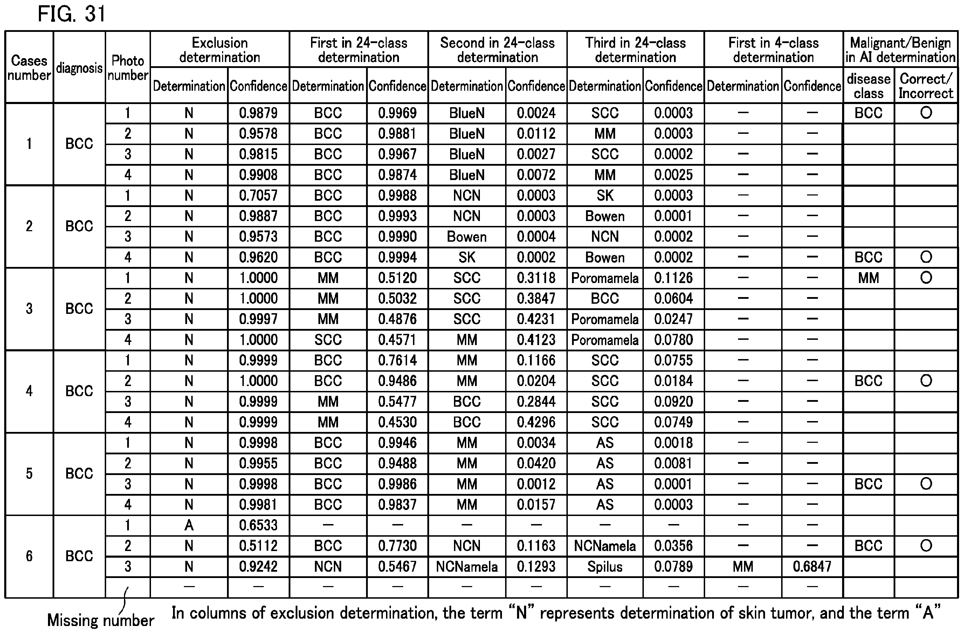

Among the photographs taken by using the client terminal, 6 cases diagnosed with BCC by a doctor were determined by the following procedures.
(1) For each case, 4 photographs were taken and sent to a server by the user terminal, and three classifiers were executed.
(2) Images determined not to be skin tumor by an exclusion determination engine were excluded from the candidates for determination. In this regard, there is one excluded image (Case number 6, Photo number 1) in Fig. 31 because the entire image was shining and the tumor was unclear. For this reason, the image is considered not to be recognized as a skin tumor.
(3) The 24-class determination was performed, and images of which the results had been SK or NCC were re-determined in 4-class.
(4) In the 24-class determination, the result with the highest confidence was used as the determination result of the present system for that case. In addition, in a case where the result with the highest confidence was SK or NCC, the result of re-determination of 4-class becomes the determination result of the present system, but there was no such a case in the 6 cases at this time.

The results are shown in Fig. 31. As shown in this Example, a system that uses several classifiers at the same time and does not overlook any malignant tumor can be constructed.

### [Realization by software]

Each process shown in Fig. 4 may be realized by a logic circuit (hardware) formed in an integrated circuit (IC chip) or the like, or may be realized by software using a CPU (central processing unit).

In a case of realizing each process by each processing software shown in Fig. 4, the user client terminal 100, the skin disease analyzer 200, and the manager client terminal 300 are provided with a CPU that executes instructions for a program being software that realizes each function, a ROM (read only memory) in which the above program and various data are recorded readable by a computer (or CPU) or a storage device (these are referred to as "recording media"), a RAM (random access memory) to develop the above program, and the like. Further, an object of the present invention is achieved when a computer (or CPU) reads the program from the recording medium and executes it. As the recording medium, a "non-temporary tangible medium" such as a tape, a disk, a card, a semiconductor memory, or a programmable logic circuit can be used. In addition, the program may be supplied to the computer via an arbitrary transmission medium (such as a communication network, or a broadcast wave) capable of transmitting the program. In this regard, one embodiment of the present invention can also be realized even in the form of a data signal embedded in a carrier wave, in which the program is embodied by electronic transmission.

The present invention is not limited to the above-described embodiments, and various modifications may be made within the disclosed range, and embodiments obtained by appropriately combining the technical means disclosed in different embodiments, respectively are also included in the technical scope of the present invention.

The present application claims a priority based on Japanese Patent Application No. 2019-067229 filed on March 29, 2019 and Japanese Patent Application No. 2019-206765 filed on November 15, 2019, and all of the contents described in these Japanese patent applications are incorporated by reference.

### Reference Signs List

- 1: Skin disease analysis system
- 2: Network
- 100: User client terminal
- 200: Skin disease analyzer
- 300: Manager client terminal

## Claims

1. A skin disease analysis program executed by a computer, to allow the computer to execute
a second step of predicting a kind of skin tumor for an image to be analyzed of skin tumor by a first-learned model that has machine learned from images of affected parts of various skin diseases in advance, and
either one or both of a first step and a third step,
wherein the first step is a step of determining whether or not the image to be analyzed is an image of skin tumor by a skin disease determination engine, prior to the second step, and
in a case where a determination result of the second step has been one kind of skin tumors that are easily mistaken for each other, the third step is a step of re-predicting the kind of skin tumor for the image to be analyzed by a second-learned model that has machine learned from images of affected parts of specific skin diseases including the skin tumors that are easily mistaken for each other.

2. A skin disease analysis program executed by a computer, to allow the computer to execute
a first step of determining whether or not an image to be analyzed is an image of skin tumor by a skin disease determination engine, and
in a case where the image to be analyzed has been determined to be an image of skin tumor by the first step, a second step of predicting the kind of skin tumor for the image to be analyzed by a learned model that has machine learned from images of affected parts of various skin diseases in advance.

3. A skin disease analysis program executed by a computer, to allow the computer to execute
a second step of predicting a kind of skin tumor for an image to be analyzed of skin tumor by a first-learned model that has machine learned from images of affected parts of various skin diseases in advance, and
in a case where a determination result of the second step has been one kind of skin tumors that are easily mistaken for each other, a third step of re-predicting the kind of skin tumor for the image to be analyzed by a second-learned model that has machine learned from images of affected parts of specific skin diseases including the skin tumors that are easily mistaken for each other.

4. The skin disease analysis program according to any one of claims 1 to 3, wherein
the second step predicts a kind of skin tumor by a first-learned model that has machine learned from images of affected parts of 4 to 50 kinds of skin tumors in advance.

5. The skin disease analysis program according to claim 1 or 3, wherein
the second step predicts a kind of skin tumor for an image to be analyzed by a first-learned model that has machine learned from 3 or more kinds of images of affected parts of skin diseases including at least malignant melanoma as the skin disease, and
in a case where the image to be analyzed has been predicted to be a tumor that is easily erroneously determined to be malignant melanoma by the second step, the third step re-predicts the kind of skin tumor for the image to be analyzed by a second-learned model that has machine learned from images of affected parts of skin diseases including malignant melanoma and a tumor that is easily erroneously determined to be malignant melanoma in advance.

6. The skin disease analysis program according to claim 1 or 3, wherein
the tumor that is easily erroneously determined to be malignant melanoma includes at least nevocellular nevus,
the second step predicts a kind of skin tumor for an image to be analyzed by a first-learned model that has machine learned from 3 or more kinds of images of affected parts of skin diseases including at least malignant melanoma and nevocellular nevus as the skin diseases, and
in a case where the image to be analyzed is determined to be nevocellular nevus by the second step, the third step is a step of re-predicting the kind of skin tumor for the image to be analyzed by a second-learned model that has machine learned from images of affected parts of skin diseases including at least malignant melanoma and nevocellular nevus in advance.

7. The skin disease analysis program according to claim 1 or 3, wherein
the second step predicts a kind of skin tumor for an image to be analyzed by a first-learned model that has machine learned from 5 or more kinds of images of affected parts of skin diseases including at least malignant melanoma, basal cell carcinoma, seborrheic keratosis, and nevocellular nevus as the skin diseases, and
in a case where the image to be analyzed has been determined to be nevocellular nevus or seborrheic keratosis by the second step, the third step re-predicts the kind of skin tumor for the image to be analyzed by a second-learned model that has machine learned from images of affected parts of skin diseases including at least malignant melanoma, basal cell carcinoma, seborrheic keratosis, and nevocellular nevus.

8. The skin disease analysis program according to claim 1 or 3, wherein
the second step predicts a kind of skin tumor for an image to be analyzed by a first-learned model that has machine learned from 5 or more kinds of images of affected parts of skin diseases including at least malignant melanoma, basal cell carcinoma, seborrheic keratosis, and nevocellular nevus as the skin diseases, and
in a case where the image to be analyzed has been determined to be malignant melanoma, basal cell carcinoma, nevocellular nevus, or seborrheic keratosis by the second step, the third step re-predicts the kind of skin tumor for the image to be analyzed by a second-learned model that has machine learned from images of affected parts of skin diseases including at least malignant melanoma, basal cell carcinoma, seborrheic keratosis, and nevocellular nevus.

9. The skin disease analysis program according to claim 1 or 3, wherein
the second step predicts a kind of skin tumor from 5 or more kinds of skin tumors including at least 4 or more kinds of malignant melanoma, basal cell carcinoma, seborrheic keratosis, and nevocellular nevus, and further including at least one kind selected from actinic keratosis, Bowen's disease, squamous cell carcinoma, poroma, sebaceous nevus, blue nevus, congenital melanocytic nevus, spitz nevus, simple lentigo, and nevus spilus, and
in a case where an image to be analyzed has been determined to be nevocellular nevus or seborrheic keratosis by the second step, the third step re-predicts the kind of skin tumor for the image to be analyzed by a second-learned model that has machine learned from at least two or more kinds of images of affected parts of skin diseases including malignant melanoma in advance.

10. The skin disease analysis program according to claim 1 or 3, wherein
the second step predicts a kind of skin tumor by a first-learned model that has machine learned from 5 or more kinds of images of affected parts of skin diseases including at least malignant melanoma, basal cell carcinoma, seborrheic keratosis, nevocellular nevus, and amelanotic poroma, and
in a case where an image to be analyzed has been determined to be amelanotic poroma by the second step, the third step re-predicts the kind of skin tumor for the image to be analyzed by a second-learned model that has machine learned from images of affected parts of basal cell carcinoma, malignant melanoma, nevocellular nevus, and seborrheic keratosis, or a second-learned model that has machine learned from images of affected parts of basal cell carcinoma, malignant melanoma, nevocellular nevus, and amelanotic poroma.

11. The skin disease analysis program according to any one of claims 5 to 10, wherein
the third step increases at least twice or more the number of images of affected parts of malignant melanoma and/or basal cell carcinoma that are similar in shape to other diseases when machine learning is performed by using the images of affected parts.

12. The skin disease analysis program according to any one of claims 1 to 11, wherein
the program allows a computer to execute a step of displaying a result of the prediction.

13. The skin disease analysis program according to claim 12, wherein
the program allows a computer to execute
a step of accepting an image to be analyzed from a user, and
a step of managing a user type of the user, and
the step of displaying a result of the prediction differentiates a display content depending on the user type.

14. The skin disease analysis program according to claim 12 or 13, wherein
the step of displaying a result of the prediction displays a predicted disease name and a coping method for the disease depending on a kind of skin tumor predicted by the second step and/or the third step.

15. The skin disease analysis program according to any one of claims 1 to 14, wherein
a prediction of whether an image to be analyzed is a malignant skin tumor or a benign skin tumor is performed.

16. The skin disease analysis program according to any one of claims 1 to 15, wherein
a prediction is performed for a plurality of images of the same affected part.

17. The skin disease analysis program according to any one of claims 1 to 16, wherein
the second step can predict that an image to be analyzed is at least one of actinic keratosis, Bowen's disease, squamous cell carcinoma, basal cell carcinoma, and malignant melanoma.

18. The skin disease analysis program according to any one of claims 1 to 16, wherein
the second step can predict that an image to be analyzed is at least one of poroma, sebaceous nevus, seborrheic keratosis, blue nevus, congenital melanocytic nevus, nevocellular nevus, spitz nevus, simple lentigo, and nevus spilus.

19. The skin disease analysis program according to any one of claims 1 to 18, wherein
information of an affected part is used for prediction, and
the information of an affected part includes at least one of age of a patient, a size of an affected part, an odor of an affected part, a site of disease development, and a three-dimensional shape of an affected part.

20. A skin disease analysis method, comprising:
a first step of determining whether or not an image to be analyzed is an image of skin tumor by a skin disease determination engine; and
in a case where the image to be analyzed has been determined to be an image of skin tumor by the first step, a second step of predicting a kind of skin tumor for the image to be analyzed by a learned model that has machine learned from images of affected parts of various skin diseases in advance.

21. A skin disease analysis method, comprising:
a second step of predicting a kind of skin tumor for an image to be analyzed by a learned model that has machine learned from images of affected parts of various skin diseases in advance; and
in a case where a determination result of the second step has been one kind of skin tumors that are easily mistaken for each other, a third step of re-predicting the kind of skin tumor for the image to be analyzed by a second-learned model that has machine learned from images of affected parts of specific skin diseases including the skin tumors that are easily mistaken for each other.

22. A skin disease analyzer, comprising:
a storage unit that stores a skin disease analysis program; and
a control unit that controls operation of a skin disease analyzer by executing the skin disease analysis program, wherein
the control unit
determines whether or not an image to be analyzed is an image of skin tumor by using a skin disease determination engine, and
in a case where the image to be analyzed has been determined to be an image of skin tumor, predicts a kind of skin tumor for the image to be analyzed by a learned model that has machine learned from images of affected parts of various skin diseases in advance.

23. A skin disease analyzer, comprising:
a storage unit that stores a skin disease analysis program; and
a control unit that controls operation of a skin disease analyzer by executing the skin disease analysis program, wherein
the control unit
predicts a kind of skin tumor for an image to be analyzed by a first-learned model that has machine learned from images of affected parts of various skin diseases in advance, and
in a case where the image to be analyzed has been one kind of skin tumors that are easily mistaken for each other, re-predicts the kind of skin tumor for the image to be analyzed by a second-learned model that has machine learned from images of affected parts of specific skin diseases including the skin tumors that are easily mistaken for each other.

24. A skin disease analysis system, comprising:
a skin disease analyzer; a user client terminal capable of transmitting an image to be analyzed to the skin disease analyzer; and a network connecting the skin disease analyzer to the user client terminal to transmit information, wherein
the skin disease analyzer has a learned model that has machine learned from images of affected parts of various skin diseases in advance, and a skin disease determination engine that determines whether or not the image is a skin disease, and
determines whether or not the image to be analyzed is an image of skin tumor by using the skin disease determination engine, and in a case where the image to be analyzed has been determined to be an image of skin tumor, the skin disease analyzer predicts a kind of skin tumor for the image to be analyzed by the learned model.

25. A skin disease analysis system, comprising:
a skin disease analyzer; a user client terminal capable of transmitting an image to be analyzed to the skin disease analyzer; and a network connecting the skin disease analyzer to the user client terminal to transmit information, wherein
the skin disease analyzer has a first-learned model that has machine learned from images of affected parts of various skin diseases in advance, and a second learned model that has machine learned from images of affected parts of specific skin diseases including skin tumors that are easily mistaken for each other, and
in a case where the image to be analyzed has been predicted to be one kind of skin tumors that are easily mistaken for each other by the first-learned model, the skin disease analyzer re-predicts the kind of skin tumor for the image to be analyzed by a second-learned model that has machine learned from images of affected parts of specific skin diseases including the skin tumors that are easily mistaken for each other.
